# EUROPEAN PATENT SPECIFICATION

(11) **EP 0 855 919 B1**
(45) Date of publication and mention of the grant of the patent: **23.07.2003**
(21) Application number: 96936786.1
(22) Date of filing: 17.10.1996
(51) Int. Cl.: A61K 48/00, A61K 31/59, A61K 47/18, A61K 47/28

(54) **INDUCTION OF MUCOSAL IMMUNE RESPONSES BY VITAMIN D3 AND ANTIGEN-ENCODING DNA COMPLEXE TO CATIONIC LIPID**
INDUKTION VON IMMUNANTWORTEN DER SCHLEIMHÄUTE DURCH VERABREICHUNG VON VITAMINE D3 UND DNS, DIE FÜR EIN ANTIGEN KODIERT UND DIE MIT MIT EINEM KATIONISCHEN LIPID EIN COMPLEX BILDET.
INDUCTION DE L'IMMUNITE MUCOSALE PAR L'ADMINISTRATION DE LA VITAMINE D3 ET D'ADN CODANT UN ANTIGENE COMPLEXE A UN LIPIDE CATIONIQUE

(30) Priority: 18.10.1995 US 544575
(43) Date of publication of application: 05.08.1998
(73) Proprietor: VANDERBILT UNIVERSITY, Nashville, TN 37240 (US)
(72) Inventor: MITCHELL, William, M., Nashville, TN 37205 (US)
(74) Representative: Price, Vincent Andrew
(86) International application number: US9616845
(87) International publication number: WO97014442

(56) References cited:
- WO-A-94/27435
- WO-A-96/40964
- ANNALS OF THE NEW YORK ACADEMY OF SCIENCES, Volume 730, issued 1994, DAYNES et al., "The Development of Effective Vaccine Adjuvants Employing Natural Regulators of T-Cell Lymphokine Production In Vivo", pages 144-161, XP000647334.
- BIOCONJUGATE CHEMISTRY, Volume 5, Number 6, issued 1994, REMY et al., "Gene Transfer with a Series of Lipophilic DNA-Binding Molecules", pages 647-654, XP000484178.
- NATURE, Volume 356, issued 12 March 1992, TANG et al., "Genetic Immunization is a Simple Method for Eliciting an Immune Response", pages 152-154, XP002044310.
- SCIENCE, Volume 265, issued 09 September 1994, SERVICE, "Triggering the First Line of Defense", pages 1522-1524, XP002941970.
- IMMUNOBIOLOGY, Volume 184, issued 1992, HOLMGREN et al., "Mucosal Immunity: Implications for Vaccine Development", pages 157-179, XP002922882.

## Description

### BACKGROUND OF THE INVENTION

### Field of the Invention.

The present invention is directed to mucosal immunity. Specifically, the invention is directed to inducing mucosal immunity in a subject. More specifically, the invention is directed to use of DNA complexed to a cationic lipid and vitamin D3 subject in the manufacture of a medicament for inducing mucosal immunity.

### Background Art.

Mucosal surfaces represent the major route of entry for most systemic pathogens with subsequent mucosal immunity usually providing long term protection against reinfection (25). Examples include the life-long immunity produced by the Sabin oral polio vaccine versus the relatively short-term protection provided by the Salk parenteral vaccine (48) and the single dose oral cholera vaccine with its improved safety profile versus the older multi-dose parenteral cholera vaccine (27). The best long-term mucosal and systemic protection against infection is provided by live, attenuated pathogens which simulate infection of the naive host but which are Incapable of inducing disease (28). Despite the current capacity to produce attenuating mutations in cloned microorganisms, the concern over potential reversion to virulence or host virulence determinants has effectively inhibited development of live attenuated pathogens as inducers of mucosal immunity for human use (29).

For example, at a recent meeting of the NIH sponsored HIV vaccine meeting in November 1994, the proponents of attenuated live virus vaccines received a blow by Ruth Ruprecht who reported that an attenuated SIV (i.e., Nef deletion) was responsible for the development of simian AIDS in newborn Rhesus macaques who had received the vaccine (29). It is unlikely that an attenuated HIV will ever receive FDA approval as an HIV vaccine.

Currently, 10,000 individuals worldwide are infected daily by the Human Immunodeficiency Virus (HIV). The World Health Organization (WHO) estimates that by the year 2000 at least 40 million people will be infected with the Human Immunodeficiency Virus (HIV). Due to the relentless and progressive pathogenesis of the virus the majority of those infected will die within 10 years. It is estimated further that the death toll will be at 10 million as we enter the 21st century. Despite an initial massive effort by industry to develop a vaccine, few commercial developers remain. NIH's National HIV Vaccine recently received a critical setback when the AIDS Research Advisory Program Committee (ARAC) voted to not proceed in Phase III clinical testing of the two leading candidate subunit vaccines.

Another difficulty with the current efforts to develop an HIV vaccine is the paucity of research in the generation of mucosal immune responses to HIV. Epidemiological data clearly indicate that 70-80% of all AIDS cases are the result of heterosexual transmission of HIV (30-38). Heterosexual transmission is the fastest growing route of transmission in the United States with women being at significantly greater risk of infection by HIV than males (39, 40). Since 90% of HIV is transmitted sexually worldwide, it is unlikely that systemic immunity will block initial infection at the mucosal sites of entry. Infection of Langerhans cells, mucosal macrophages, T cells, and even epithelial cells from cell associated HIV or free HIV in semen of the genital tract strongly suggests that the induction of mucosal responses are at least as important as systemic responses in the development of a vaccine against HIV infection (35-37,41). Although systemic immunization rarely induces mucosal immunity, mucosal immunization frequently provides systemic responses as well (36, 41, 42, 43, 44, 45, 46). It is essential that more effort be devoted to this key element in establishing a primary defense against HIV transmission. With the clear danger of using live attenuated virus, the prospects for inducing mucosal immunity are difficult.

Recent developments in vaccine research include the demonstration that transfection of mouse muscle with a bacterial plasmid carrying the DNA sequence encoding an influenza virus nucleoprotein resulted in the development of humoral and cellular response which protected mice from lethal viral challenge (1). This follows the observation that mouse muscle is a unique target for transfection with naked DNA (3) and that muscle of a variety of species is particularly susceptible to naked DNA transfection (4-11). Protection against lethal challenge in mice by influenza A virus, and induction of cytotoxic lymphocytes and neutralizing antibodies to influenza A virus (1, 13-15) and HIV (16, 17), following genetic IM immunization has been reported by a number of investigators. This method has the disadvantage that relatively massive quantities of DNA are required. Although unreported as a toxic side effect to date, this requirement for large quantities of DNA may limit this method due to the potential for antibody response to DNA itself and the generation of a self sustaining lupus-like syndrome. More importantly, despite the impressive induction of protective immune responses, this method does not induce mucosal immunity. A less common approach to genetic immunization using bolistic transformation overcomes the problem of DNA quantity but requires instrumentation not widely available. Typically, nanogram quantities of DNA complexed to gold or tungsten particles are physically propelled through the plasma membrane by microprojectile bombardment. While both methods elicit cellular (21, 22) and humoral responses (22-24), neither induces mucosal immunity.

Despite the importance of mucosal immunity for an effective immunization strategy, the only FDA approved vaccine that induces mucosal immunity is the Sabin, live-attenuated oral polio vaccine. More recently, another development in the generation of mucosal immunity was the demonstration that the systemic administration of activated vitamin D3 (1,25-dihydroxycalciferol [1,25(OH)₂D3]) with a conventional protein antigen triggers a mucosal response in addition to systemic immunity(2). Thus, the art is actively seeking ways to induce a mucosal immune response.

The present invention meets a very important need in vaccine production by providing a composition for inducing *in vivo* mucosal immune responses to antigens of pathogens by the facilitated transfection of mucosa with a bacterial plasmid carrying the DNA sequence for the antigen.

### SUMMARY OF THE INVENTION

The invention provides use of vitamin D3 and antigen-encoding DNA effective to induce a mucosal immune response complexed to a transfection-facilitating cationic lipid in the manufacture of a medicament for inducing a mucosal immune response. The invention also provides use of antigen-encoding DNA effective to induce a mucosal immune response, complexed to a transfection-facilitating cationic lipid (e.g., lipospermine/lipospermidine, TEDBI, etc.), and an amount of 1,25(OH)₂D3 in the manufacture of a medicament inducing for a mucosal immune response. The antigen-encoding DNA can encode an antigen that is expressed on the surface of infected cells during the course of infection or is a surface attachment protein of the pathogen. The present invention should apply to all mucosally acquired pathogens in which receptors on mucosal cells recognize proteins of the pathogen providing an attachment site for entry systemically (i.e., most bacterial pathogens) or to actively gain intracellular entrance to target cells (i.e., viruses and some bacterial pathogens). DNA encoding the envelope glycoproteins of viral pathogens is a rational choice for use in the present method.

Cationic lipids are a class of charged lipids capable of complexing with DNA by charge-charge interactions with its phosphate backbone. The lipid facilitates penetration of the plasma membrane of eukaryotic cells. Examples include lipospermines and lipospermidines, which are bifunctional molecules consisting of one or more hydrophobic chains covalently linked to a cationic grouping in which there is a coordination of three or more amide hydrogens with a phosphate oxygen of the DNA chain forming an ionic charge complex. One preferred example of a lipospermine is DOGS (dioctadecylamidoglycylspermine), Dioctadecylamidoglycylspermidine is another likely candidate, because it has the same structure as DOGS, but lacks one of the two arms having two non-essential cationic charges. Another example is N,N,N',N'-tetramethyl N,N'-bis(2-hydroxyethyl)-2,3-dioleoyloxy-1,4-butanediammoniun iodide (TEDBI) (tfx™-50 Reagent; Promega, Madison, WI). This cationic lipid also facilitates transfection *in vitro* and *in vivo*.

The invention also provides a composition, comprising vitamin D3 and an antigen-encoding DNA, wherein the DNA is complexed to a transfection-facilitating cationic lipid. Preferably the DNA encodes an envelope antigen or envelope-associated antigen of a pathogen and the cationic lipid is lipospermine, lipospermidine or DOGS.

### DESCRIPTION OF THE FIGURES

Fig. 1 shows a circular map of pHenv showing HIVenv insert between 5' and 3' LTRs. Rev is functional in this construct.
Fig. 2 shows a circular map of pCMV-env160 containing HIVenv under a CMV promoter and lacking LTR's or rev sequences.

### DETAILED DESCRIPTION OF THE INVENTION

The invention provides use of vitamin D3 and an antigen-encoding DNA effective to induce a mucosal immune response complexed to a transfection-facilitating cationic lipid in the manufacture of a medicament for inducing a mucosal immune response. The invention also provides use of antigen-encoding DNA effective to induce a mucosal immune response, complexed to a transfection-facilitating cationic lipid, and an amount of 1,25(OH)₂D3 in the manufacture of a medicament of produce a mucosal response.

The invention is applicable to pathogens generally, because expression of the pathogen antigen encoded by the antigen-encoding DNA results in exposure of the pathogen antigen on the surface of the transfected cell, mimicking either a portion of the replicative cycle of the pathogen or expressing antigens required for the initial attachment of the pathogen to the cell surface, Examples of viral pathogens include, but are not limited to, retroviruses (human immunodeficiency viruses), herpesviruses (herpes simplex virus; Epstein Barr virus; varicella zoster virus), orthomyxoviruses (influenza), paramyxoviruses (measles virus; mumps virus; respiratory syncytial virus), picornaviruses (Coxsackie viruses; rhinoviruses), hepatitis viruses (hepatitis C), bunyaviruses (hantavirus; Rift Valley fever virus), arenaviruses (Lassa fever virus), flaviviruses (dengue fever virus; yellow fever virus; chikungunya virus) and coronaviruses, among others. Examples of bacterial pathogens include, but are not limited to, species of the following genera: *Salmonella, Shigella, Chlamydia, Helicobacter, Yersinia, Bordatella, Pseudomonas, Neisseria, Vibrio* and *Haemophilus*, among others.

### Antigen-Encoding DNA

The present invention can be applied to all mucosally acquired pathogens in which expression of antigen on the surface of a mucosal cell occurs during the course of natural infection or in which a surface antigen is required for attachment to mucosal cells. In a method of inducing a mucosal immune response, the antigen-encoding DNA can encode an antigen that is expressed on the surface of infected cells during the course of infection or is required for attachment to target cells of the host. The present method will result in the antigen encoded by the DNA being expressed on the surface of mucosal cells, where a mucosal immune response will develop or the hormonal induction of mucosal immunity from systemic transfected cells (e.g., muscle). Because the primary immune response to bacteria is to a relatively small number of cell surface antigens, the process for selecting antigen-encoding DNA for bacterial pathogens for use in the present method is routine. For example, the major bacterial immunogens are epitopes on exposed bacterial surface structures, which serve to attach the bacterium to mucosal cells during infection (191). There are numerous examples of viral antigens in which this is the case, for example, HIV envelope to CD4 bearing cells. Examples of other such antigens are described in virology textbooks (see for example *Fundamental Virology*, 2nd. E., pp.373-375 (189)). Antigens of other microbial pathogens are expressed as epitopes on cell surface structures. As used herein, an "antigen" is a molecule that elicits an immune response and is used interchangeably with "immunogen".

DNA encoding the envelope glycoproteins (*e.g*., gp160 HIV or its cleaved derivative proteins, gp41 and gp120) of viral pathogens is one rational choice for use in the present invention. Envelope-associated proteins, such as gp17 are also reasonable choices, because of their presentation on the cell surface of infected cells. A reasonable terminology to define a subset of viral antigens that will be effective in this method is "envelope and envelope-associated proteins." Specific epitopes of these proteins that elicit an immune response in a subject can be selected by routine methods, including epitope mapping and analysis of conformational dependency (178). Particularly, epitopes that elicit neutralizing antibodies are important bases of the present method. Epitopes eliciting neutralizing antibodies can be selected by routine methods, including induction of monoclonal antibodies to specific epitopes coupled with analysis for specific neutralization in standard dose dependent assays (178). DNA encoding these antigens can be obtained by cloning and synthesis methods known in the art and. further described below.

For example, the antigen-encoding DNA can encode an antigen of a human immunodeficiency virus. As a more specific example, the antigen-encoding DNA can encode a human immunodeficiency virus envelope glycoprotein. Although the envelope antigens are expected to be the main inducers of antibodies and cytotoxic lymphocytes (CTLs), there is literature evidence of CTLs against the gag (*i.e*. internal antigen) of HIV. Antigen-encoding DNAs include gp160, gp120 and gp41 separately expressed (*i.e*., gp160 is normally cleaved by a host protease to gp120 and gp41). DNA encoding gp17, which is one of the gag proteins that is attached by a myristylation link to the envelope, and for which there is literature evidence for a neutralizing antibody epitope close to the myristylation site, can also be included. The antigen-encoding DNA can encode antigenic fragments of the envelope and envelope-associated proteins, for example, the V3 loop of a human immunodeficiency virus envelope glycoprotein.

An antigen-encoding DMA should have a start codon, a signal peptide, a stop codon and should have a membrane anchor. A secreted antigen (lacking a membrane anchor) will elicit an immune response, but is not expected to be as effective. Thus, if these are not present, or in order to optimize the present method, the sequences of antigen-encoding DNA can be mutated in one or more ways to preserve or enhance the antigenicity of the expressed antigen. If separate gp120 and gp41 immunogens are used, each should have a membrane anchor followed by a stop codon. Thus, a stop signal can be generated for gp120 as well as a membrane anchor at the C-terminal region of the translated protein. In addition, the known antibody enhancing domain of gp41 can be removed for both HIV and RSV as described in detail in the Examples. Numerous versions of the V3 region of the envelope glycoprotein can be made to reflect the major quasispecies found in viral isolates. For, example numerous HIV variants have been isolated and sequenced as described in the literature. These can then be administered in multiple genetic constructs, each containing a single transcribed ORF, or in a single or a few genetic constructs, each containing multiple transcribed ORFs. Genetic manipulations of this nature are known in the art (188) and specific examples described in the Examples.

Briefly, mutations can be produced using the p-Alter-1 kit from Promega, which incorporates antibiotic selection for selection of the desired mutations. It is necessary to use the ssDNA template procedure for reliable generation of desired mutations. A critical change from the kit protocol is the generation of the presently taught helper phage ssDNA. The ss Phage DNA isolation kit and procedure from Biolabs, Inc. is used for the production of pure ssDNA. Substitution of the Es 1301 mutS *E. coli* supplied with the kit with XL mutS *E. coli* for which the subject mutS mutations have been generated has also been successful. The latter are devoid of repair enzymes. The components of the above method are generally applicable to DNA encoding other antigens.

Examples of gene engineering that are expected to be incorporated into a plasmid containing, for example, the HIV envelope for transfection of eukaryote cells and antigen expression on the cell surface include: 1) elimination of the HIV LTR control elements and placement under a more powerful promoter such as CMV, 2) elimination of the gp160 proteolytic cleavage site so that gp120 does not disassociate from the membrane anchored gp41 and 4) the elimination of the primary enhancing domain by point or deletion mutations which destroy this capacity. For example, an in frame deletion of nt 1516 to nt 1527 (i.e., RDKR) *results* in a gp160 lacking the proteolytic cleavage site at the arginine-alanine sequence at residues 508-509 where proteolysis occurs. Further examples of these mutations are further described below. Although, specific mutations for HIV envelope glycoproteins are given, it is understood that the same considerations for the generation of an efficient immunization construct apply to the generation of a construct using an antigen encoding DNA for a different antigen.

The vectors used in the present invention can include promoters and regulatory sequences that are relevant to the antigen-encoding DMA. Typically, the vector must be a eukaryotic vector that is capable of replication in *E.* *coli*. The preferred vector contains a bacterial origin of replication, an antibiotic resistance selection gene, eukaryotic promoter, the gene to be transcribed in a eukaryotic cell and a polyadenylacion gene for efficient translation. Descriptions of vectors having these characteristics are common in the literature. Other vectors can be designed by the skilled artisan that do not share all of the above characteristics, yet permit transfection.

### Cationic Lipids

The transfection-facilitating cationic lipids used in the invention are bifunctional molecules consisting of one or more hydrophobic chains covalently linked to a cationic grouping in which there is a cationic charge complex formed between the negative charge on a phosphate oxygen and one or more positive charges on the cationic lipid. In the case of DOGS there is coordination of three or more amide hydrogens with a phosphate oxygen of the DNA chain forming an ionic charge complex. To facilitate transfection, the cationic lipid can both protect the DNA, neutralize its negative net charge, and make it appear more hydrophobic to the cell membrane of the cell to be transfected. For example, the charge interaction positions the hydrophobic arms along the major or minor groove of DNA (see Examples) providing a hydrophobic covering for the highly charged DNA macromolecule and affords facilitated cellular entry by association of the hydrophobic surface covering DNA with the hydrophobic component of the plasma membrane of the cell. Based upon molecular modeling using DOGS, it appears that the amino hydrogen of the peptide bond and adjacent amide hydrogens all coordinate on one phosphate oxygen (*i.e*., 1.91 to 2.0 Å distance).

One preferred example of a lipospermine is DOGS (dioctadecylamidoglycylspermine). Although less preferred, the lipospermine or lipospermidine could have a single hydrophobic side chain (*e.g*., monooctyl, monooctadecyl, monododecyl, etc.). The nature of the charge group can also be modified as can the saturation of the hydrophobic side chains as with TEDBI.

A possible example of molar cationic ratio of DOGS to DNA is about 5:1. Alternatively, this lipospermine can be complexed to DNA in a molar cationic ratio ranging from about 2 to about 10. Because the ionic interactions between the cationic lipid and the DMA is the same regardless of the antigen encoded, the present teaching with regard to the formulation of DNA-cationic lipid complexes is applicable to any antigen-encoding DNA.

Thus, the invention also provides a composition, comprising vitamin D3 and an amount of DNA encoding an envelope antigen or envelope-associated antigen of a pathogen complexed to a lipospermine. More specifically, the invention provides a composition, comprising viatamin D3 an amount of DNA encoding an envelope antigen of HIV complexed to a cationic lipid. For instance, the composition can comprise a plasmid described in the Examples. Other examples of antigen-encoding DNA are described herein and in the literature.

### Hormone Immunomodulation

The present invention utilizes vitamin D3 in conjunction with facilitated transfection using DNA and a cationic lipid. The combination of vitamin D3 and cationic lipid-complexed DNA results in a mucosal immune response even when administered intramuscularly.

The vitamin D3 can be 1,25(OH)₂D3 or the unhydroxylated form. The unhydroxylated form must be converted in the liver and kidneys to the hydroxylated (activated) form in order to induce mucosal immunity The amounts of activated vitamin D3 can be as described in the examples. Clearly, these amounts can be adapted to a particular administration protocol or a particular subject as well as to the other components in the vaccine. Because the use of unhydroxylated vitamin D3 is less efficient, significantly larger quantities will be needed. Optimization of dosages is a routine aspect of immunization protocols.

### The Mucosal Immune System

Significant indirect evidence indicates the presence of a common mucosal immune system (47,50). Induction of mucosal immunity in bronchus-associated lymphoid tissues usually yields evidence of immunity in gut-associated lymphoid tissues. The common element is the generation of mobile IgA secreting plasma cells with an affinity for mucosal-associated lymphoid tissues of various types (See Reference 42). Although IgG can be found on mucosal surfaces following mucosal immunizations, IgA is the predominant Ig in mucosal immunity. This is secondary to the presence of an Ig receptor with greatest affinity for polymeric (p)IgA. This receptor is expressed on the surface of mucosal epithelial cells and actively transports pIgA to the mucosal surface (47-49) through mucosal epithelial cells.

Protective immunity is determined first using animal models for the relevant pathogens. Although protective immunity may be inferred by a vigorous humoral and/or cellular immune response to an administered vaccine candidate, demonstration of the protective capacity should be demonstrated in animal models where possible. For example, mice can be infected with *Chlamydia*. Thus, the present method using DNA encoding a *Chlamydia* antigen can be used to immunize mice, which can subsequently be challenged with the bacteria to demonstrate efficacy of the candidate vaccine. Other pathogens, however, are more difficult to demonstrate protective utility prior to human clinical trials due to the lack of a suitable surrogate animal model. For example, the only accepted animal surrogate for HIV-1 infection is the chimpanzee which is an endangered species. SIV can be used as an alternative for HIV-1 in rhesus macaque. Nevertheless, significant nucleotide sequence differences exist between HIV- 1 and SIV which make direct extrapolation to human infection with HIV-1 less than ideal. Similarly, some species differences in immune responses can be expected. Nevertheless, the mechanism of action of the present method is generally applicable to other hosts and pathogens. Thus, results with other animals and other pathogens are expected to be similar. In any case, these animal tests are routine given the present teaching of the immunization protocol.

### Administration

In a method of inducing a mucosal immune response by direct application to the cells of the mucosa, the antigen-encoding DMA in complex with a cationic lipid is delivered to the mucosa of the subject. The administration can be directly to the mucosa, in which case specific examples of the mucosal administration include nasal, oral, rectal and vaginal. Nasal administration can be by nasal lavage spray (see Examples) or nebulizer among well practiced methods. Rectal, vaginal, vulvar or perineal administration can be by a variety of methods, including lavage (douches, enemas, etc.), suppositories, creams, gels, etc.

Systemic administration can also be used to trigger specific immune responses in the mucosa of the subject. Particularly effective is intramuscular injection of vitamin D3 with the DNA and other components of the vaccine formula as described in detail in the Examples.

Depending on the intended mode of administration, the compounds of the present invention can be in pharmaceutical compositions in the form of solid, semisolid or liquid dosage forms, such as, for example, tablets, suppositories, pills, capsules, powders, liquids, suspensions, lotions, creams, gels, or the like, preferably in unit dosage form suitable for single administration of a precise dosage. The compositions can include, as noted above, an effective amount of the DNA and, in addition, may include other pharmaceutically acceptable medicinal agents, pharmaceutical agents, carriers, adjuvants, diluents, etc. By "pharmaceutically acceptable" is means a material that is not biologically or otherwise undesirable, *i.e.*, the material may be administered to an individual along with the antigen-encoding DNA without causing any undesirable biological effects or interacting in a deleterious manner with any of the other components of the pharmaceutical composition in which it is contained.

For oral administration, large doses of complexed DNA with vitamin D3 can be given. The form can be fine powders or granules may contain diluting, dispersing, and/or surface active agents, and may be presented in water or in a syrup, in capsules or sachets in the dry state, or in a nonaqueous solution or suspension wherein suspending agents may be included, in tablets wherein binders and lubricants may be included, or in a suspension in water or a syrup. Where desirable or necessary, flavoring, preserving, suspending, thickening, or emulsifying agents may be included. Tablets and granules are preferred oral administration forms, and these may be coated. Actual methods of preparing such dosage forms are known, or will be apparent, to those skilled in this art for example, see *Remington's Pharmaceutical Sciences* (190).

In the invention, the DNA is complexed to cationic lipid and is administered to the subject as a single primary vaccination, and may be followed by one or more booster vaccinations at three week to three month intervals. The booster vaccination can be by the same or by a different mode as the primary vaccination. For example, a primary intramuscular administration with activated vitamin D3 can be followed by mucosal administration of the booster with or without vitamin D3. Optimisation of the primary/booster administration regimen can be made using widely known and routine optimization procedures.

The exact amount of DNA required can vary from subject to subject, depending on the age, weight and general condition of the subject, the particular formulation used, its mode of administration, and the like. Thus, it is not possible to specify an exact amount. However, an effective amount may be determined by one of ordinary skill in the art using only routine experimentation given the teachings herein. Thus, the amount of DNA administered can be any effective amount. There no reason to expect more that minor differences in a human immunizing dose vs. mouse dose, because there is no reason to expect that human cells are more or less susceptible to transfection than mouse cells. Typically, the preferred amount of DNA required for effective transfection is from about 10 ng to 10 µg. Variations in the transfection efficiency between humans and mice can be accommodated by routine adjustments in the dosage. For example, the amount can range from 1.0 ng to 1 mg. Anything over 10µg DNA becomes logistically difficult to handle, increases the risk of toxicity and is impractical. The amount of 1,25(OH)D3 typically will range from 10 ng to 10 µg and administered IM with the cationic lipid/DNA complex.

The following examples are intended to illustrate, but not limit, the invention. While the protocols descried are applied in the context of HIV immunization, they have applicability with other pathogens that have mucosal infection mechanisms. While the protocols described are typical of those that might be used, other procedures known to those skilled in the art may be alternatively employed.

### EXAMPLES

### CATIONIC LIPID FACILITATED GENETIC IMMUNIZATION

### Genetic Immunization

The ability to simulate viral replication by transfection of non-replicating, transcription/translation-permissive viral DNA encoding viral proteins essential for a protective immune response by the host provides the advantages of an attenuated, live vaccine without the potential for reversion to virulence.

Genetic immunization offers unique advantages to the vaccine field. DNA is easy to prepare and manipulate. A variety of eukaryotic promoters, signal sequences, and hydrophobic anchors can be constructed to maximize immune responses. Advantageous site-directed mutations are relatively easy to achieve. DNA is stable and requires no refrigeration in the field during mass population vaccinations. Genetic immunization produces both humoral and cellular immune responses similar to attenuated microorganisms. The most important advantage for an HIV vaccine, however, is the relatively easy formulation of multiple sequence variations in a single genetic immunization, each of which will normally be expressed on the cell surface with the development of a wide repertoire of protective responses. The major disadvantage has been the relative massive quantities of DNA required.

### HIV Phenotypic Expression

Primary infection of a naive host by HIV-1 results in a variable clinical course (177). In the majority of cases (50-70%) an acute clinical syndrome of malaise and fever lasting 1-2 weeks associated with viremia occurs some 2-4 weeks following exposure (163-164). In a minority of patients this acute phase of infection is subclinical. Although occasional primary infections progress to AIDS very rapidly (65), most patients enter an symptomatic phase with a subsequent variable progression to AIDS after 1 to more than 10 years (166). Presumably, this initial viremic phase is controlled by an effective immune response against the initial infecting viral genotype (171). If the initial viremic phase is initially controlled by hose defense mechanisms, the question remains why the virus eventually gains the upper hand over the initially effective humoral and cellular defense mechanism.

One of the striking characteristics of HIV is its mutability especially in the viral envelope of glycoproteins, gp120 and gp41. The high mutation rate in HIV is believed to be a function of the high error rate by reverse transcriptase in the conversion of viral 70S ss RNA into proviral DNA (estimated at 2 errors per viral copy). This high mutation rate is suggestive of a potential for phenotypic expression variants which may explain, in part, the high degree of interindividual variability to HIV infection.

A preponderance of data indicates that most primary infections by HIV-1 have a non-syncytial inducing (NSI phenotype (*i.e*., monocytotrophic virus unable to form syncytia in allogenic primary co-culture or in T-cell indicator cells) in contrast to the syncytial inducing (SI) phenotype found with increasing frequency with disease progression (163-170). Ho and his colleagues (171) have carefully examined specific sequences from gp120, gp41, nef, and p17 using cloned PCR amplified DNA from PBMCs of five seroconvertors and two sexual partners. The HIV transmitters exhibited substantial HIV sequence heterogeneity while there was marked HIV sequence homogeneity from the recent seroconvertors which corresponded to minor species in the transmitters. Surprisingly gp120 exhibited the greatest sequence homogeneity (> 99% similarity). These data suggest that HIV infection is much more sequence restricted at least for sexual transmission than ever considered previously. Secondly, they suggest that the phenotypic expression of acquired genomic variation is responsible at least in part for the varied clinical progression of HIV disease although secondary HIV infection cannot be excluded as the source of genomic variability during disease progression.

One expression of acquired genomic variation is the conversion of primary HIV-1 isolates from NSI, monocytotropic to SI, T-cell line permissive variants (*i.e*., virtually all isolates can replicate in PBMCs but only SI isolates replicate in T-cell lines). The primary determinant for this functional (NSV vs SI) tropism (monocytotropic vs T-cell line permissive) is the third variable domain (V3 loop) of gp120, a glycoprotein of HIV-1 which is fully exposed on the viral surface (172). The V3 loop is a disulfide-linked polypeptide composed of 34-37 amino acids with a conserved tetrad GPGR motif midway in its sequence (173). The remainder of the sequence is highly variable and has been identified as a fusion domain of gp120 (55). Another lab (175, 176) has demonstrated that amino acid sequence changes in the V3 loop which reduced cleavage by several serine proteases conferred the NSI functional phenotype on viral recombinant vaccinia viruses expressing HIV-1 envelope sequences. These V3 loop amino acid variations determining SI vs NSI functional phenotype are illustrated in Table 1.

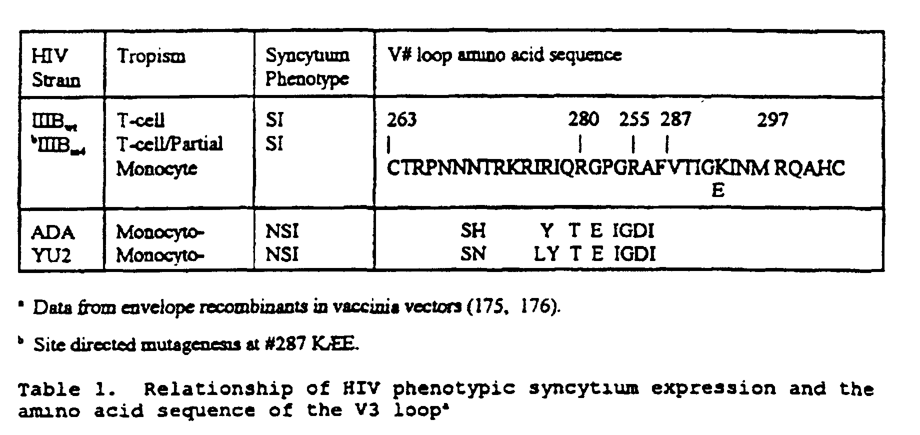

### Functional Immune Responses to HIV

An important concept that is frequently dismissed in considering the design of an HIV vaccine is that both protective and adverse immune responses can be generated by the virus or its envelope component.

### 1. Neutralization of HIV

A number of HIV-neutralizing epitopes (an epitope being defined as the minimum number of amino acid residues, either linear or conformational, that can be bound by an antibody) or domains (regions containing a cluster of epitopes) have been identified, including one within the p17 gag protein (51) and many within the gp160 envelope protein. These domains are summarized in Table 2 with respect to the specific sequence identified (amino acid residues within designated Peptides being numbered according to the Los Alamos database (52), the specificity of the neutralizing response, the relative immunogenicity of the domain, and the role of these antibodies in blocking CD4 receptor binding. Several domains have been identified by immunizing animals with synthetic peptides and testing the hyperimmune serum for the ability to neutralize HIV-1 *in vitro*. By this method, residues 247-267 (53), 296-331 (54-59), 451-477 (54) and 496-525 (60) within gp120, and residues 593-604 (61), 609-625 (54) and 721-745 (54,60,62) within gp41 have all been reported to stimulate the production of HIV-neutralizing antibodies in experimental animals. However, investigators have had some difficulty in determining what constitutes a significant neutralizing response, since several of these peptides only stimulated antibodies that could neutralize HIV-1 to a titer of 1:4 or 1:8 (54). Similarly, the antibody effect on gp120 binding to the CD4 receptor is of significant interest. Only two domains have been examined, however. Antibodies to the second conserved domain (domain 1 of Table 2) have no effect on binding (53), while antibodies to the recognized CD4 receptor-binding domain effectively inhibit binding. (63).

There is also evidence confirming the presence of antibodies that neutralize HIV in the serum from HIV-infected people and chimpanzees. The vast majority of reports concern antibody to the V3 loop (residues 296-331) (57, 58, 64, 65). These antibodies have been shown to be responsible for the type-specific neutralizing response to HIV-1. Type-specific antibodies neutralize one strain (*i.e.*, HIV_{IIIB}, HIV_{RF}, etc.) while group-specific antibodies neutralize more than one strain. This region is hypervariable yet contains a highly conserved Arg-Gly-Pro-Gly-Arg sequence at residues 311-316 (66). Immune responses to the V3 loop are complicated by the hypervariable sides of the loop (residues 296-309 and 317-333). It seems that much of the antibody to the loop is concentrated against the hypervariable regions, and therefore the antibody response to the loop is type-specific (67-71). HIV_{MN} is the most universally recognized strain of HIV in North America with respect to the frequency of HIV-infected subjects with neutralizing activity towards HIV_{MN} and the geometric mean titer of all sera against HIVₘₙ (72). Although the dominant antibody response is type-specific against linear epitopes, there may be some group-specific neutralizing response to the HIV_{MN} V3 loop, perhaps to conformational epitopes involving the conserved sequence at residues 311-315. Using her extensive repertoire of human MAbs to the V3 loop, Zolla-Pazner convincingly argues for conformational influence on binding to infectious virus. Her data blunt the distinction between group- and type-specific neutralization. The role of the V3 loop antibodies in HIV infection is discussed in greater detail later. One disturbing complication with the V3 findings is the emergence of anti-V3-loop-resistant viruses following *in vitro* treatment of HIV with neutralizing anti-V3-loop monocolonal or polyclonal antibody. Mutations can occur both within (73-76) and outside the V3 loop (77,78). Indeed, it has been shown that HIV-infected subjects can develop variants that resist previous isolate-specific neutralization (79). One non-V3-loop mutant has been sequenced, and the only change in the amino acid sequence of the envelope glycoprotein was a substitution of threonine for alanine at residue 582 (78), a region not only outside the V3 loop but residing in the amino-terminal region of gp41. It has been convincingly shown that this immune-selected point mutation is not part of a specific neutralization epitope (80). Therefore, other regions of the envelope may interact with the V3 loop, thus complicating the development of a vaccine. *In vivo* neutralization-escape mutants have also been described in HIV-infected chimpanzees (81), where non V3-loop mutations were responsible for the escape from HIV-neutralizing antibodies. Group-specific neutralization of HIV infection has been demonstrated in several laboratories (82-86). These group-specific antibodies may block infection via CD4 or some alternative HI receptor (87-89).

It is recognized with regard to neutralizing antibody domains that relative immunogenicity as a result of natural infection, versus the experimental induction via synthetic peptides linked to a carrier, is frequently divergent (Table 2). The immunodominant regions of gp120 and gp41, which induce large quantities of antibody, are relatively weak as experimental immunogens, while those of gp120 that induce relatively little antibody during a natural infection are strong inducers of antibody when coupled to a carrier. This suggests the presence of alternative routes of antigen processing between infection and that produced by recombinant viral proteins or synthetic peptide immunogens, which may be important in the design of vaccines. However, since the present genetic mucosal immunization mimics viral infection, functional immune responses should accurately reflect those from *de novo* HIV infection.

The identification of neutralizing domains has also been made easier by the production of monocolonal antibodies (MAbs) against the HIV. Several anti-V3 loop MAbs have been produced that neutralize a specific virus isolate (91,92,108,109) as well as one MAb which also mediates cellular cytotoxicity (90). These include a number of murine MAbs (mu-MAbs) (108,109) and several human MAbs (hu-MAbs) (91,92). Several neutralizing monoclonal antibodies to other regions of the HIV envelope have also been described, including amino acid residues 423-437 (63), residues 728-745 (186) and the CD4+ binding domain (91). Several additional neutralizing MAbs have been shown to bind to HIV envelope glycoproteins (92,93). One of four MAbs had neutralizing activity and bound to gp41 (92). Furthermore, a report by Hansen et al. (94) indicates that MAbs directed against three different carbohydrate moieties, either N- or O-linked, were able to neutralize both HIV_{IIIB} or a patient isolate *in vitro.* A study by Mueller *et al*. (95) demonstrated that polyclonal antiserum against yeast mannan inhibited HIV replication. The importance of virus glycosylation in HIV infectivity has been reported previously by several laboratories (96-106). Thus, simple inhibition of functional glycosyl groups could explain the neutralization effects by antiglycosyl antibodies (94,95). Other data, however, suggest that secondary and tertiary structures of the envelope glycoproteins are of significant importance in the generation of group-specific rather than type-specific neutralizing antibodies. A requirement for carbohydrate in the group-specific neutralization of HIV has been demonstrated by comparing antibodies raised against a glycosylated versus a non-glycosylated gp120 (107), and by comparing the specificity of neutralization by serum eluted from non-glycosylated gp120 (108). In both cases carbohydrate was required for group-specific but not type-specific neutralization of HIV. Moreover, a recent report demonstrates that elimination of all five variable regions with retention of disulfide bonds in a non-glycosylated recombinant HIV produces an immunogen that is incapable of generating neutralizing antibodies (107). Thus, it is not known whether antibodies block specific function by blocking carbohydrate binding or whether they inhibit by disrupting native secondary and tertiary conformations required for infectivity. The locations of N-linked carbohydrate structures were studied with respect to their linear relationship to known functional antibody domains on gp1 and gp41 as well as epitopes recognized by CD8+ and CD4+ CTLs from HIV seronegative rHIV vaccines (20).

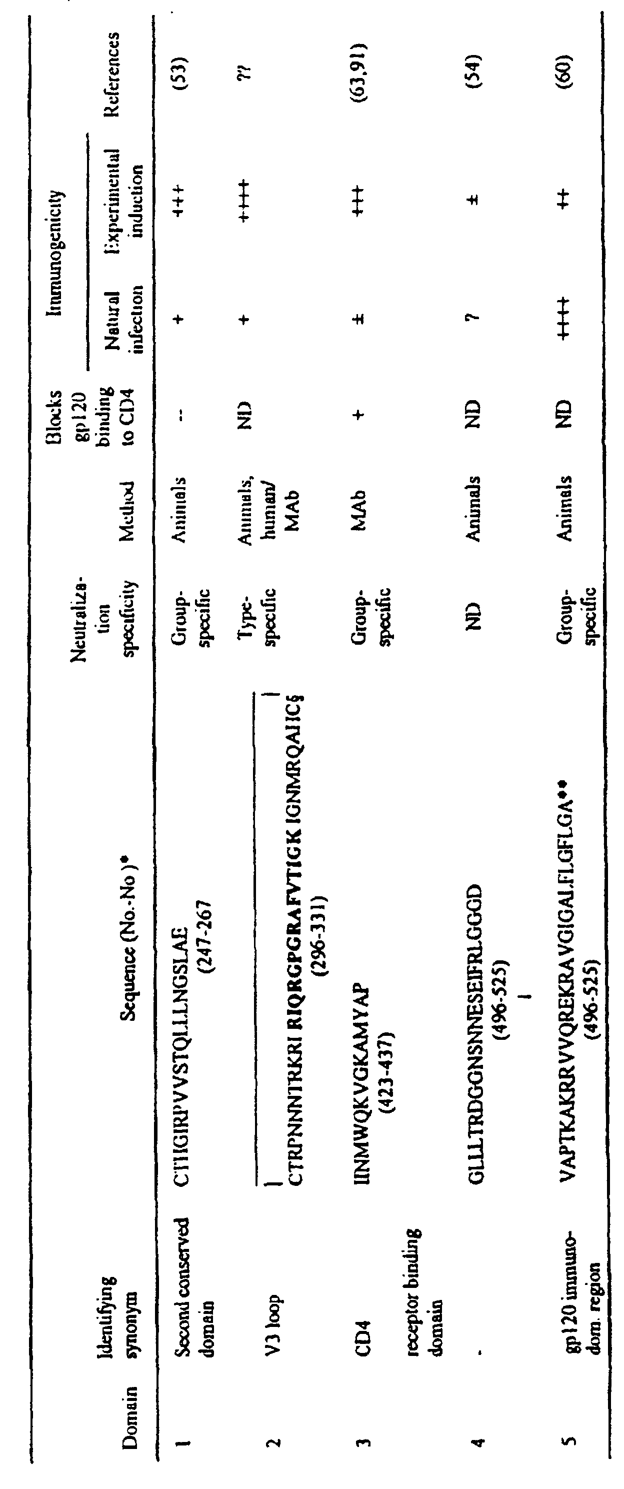

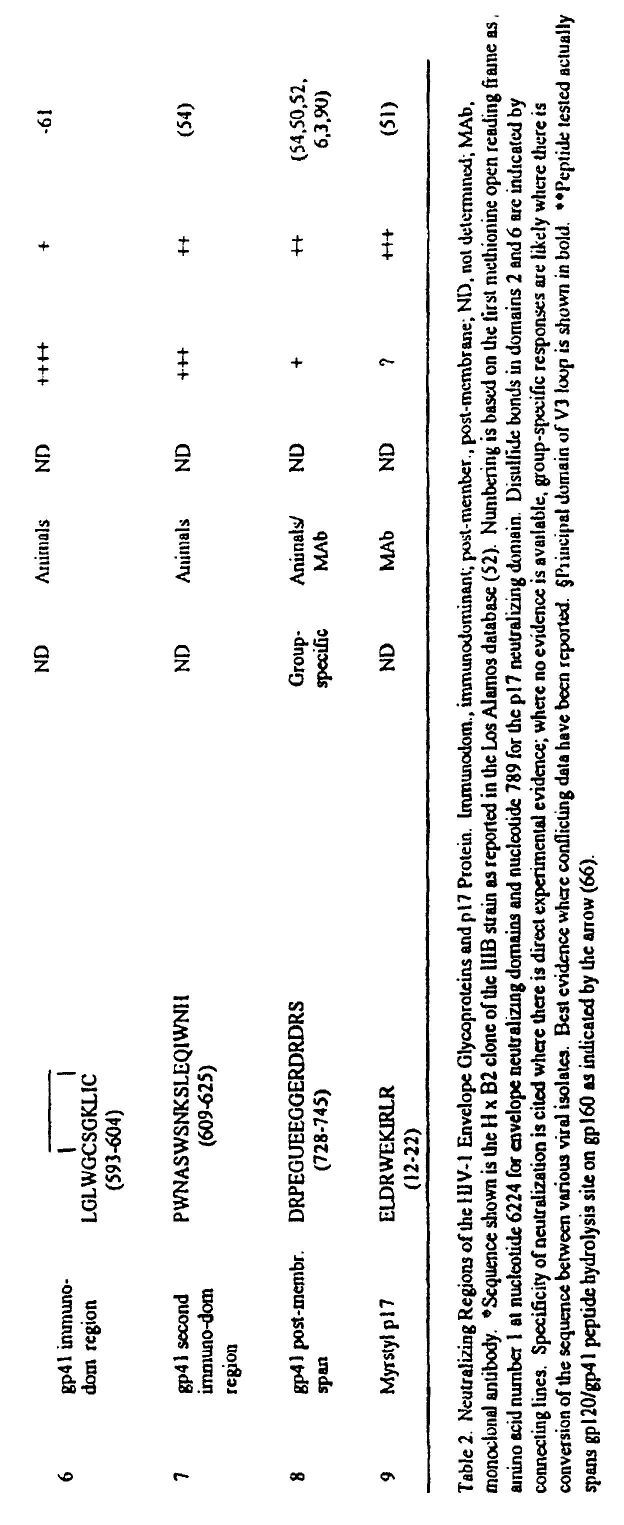

### 2. Antibody-dependent enhancement of HIV

Antibodies that enhance viral infectivity have been described for a number of viruses (112-126). The most frequently cited example involves enhancement of dengue virus infection (112-115). Results indicate that non-neutralizing antibodies can actually increase the number of infectious virions *in vitro* by binding virus to Fc receptors on monocytes and macrophages (118). In dengue infection, the degree of enhancing antibody present roughly correlates with disease severity (113). In addition to this Fc receptor-mediated mechanism, it has been shown that enhancement of infection by a flavivirus, West Nile virus, can be mediated by complement and complement receptors on cells (119). Enhancement has been demonstrated *in vitro* for a number of viruses including flaviviruses (116- 122), alphaviruses (123), rabies virus (124), Sinbdis virus (125), and coronavirus (126). There is some evidence for *in vivo* enhancement of several other viruses where ineffective vaccination resulted in increased severity of disease. The most notable of these examples occurred in children immunized against respiratory syncytial virus (RSV) (127-130) or cotton rats immunized against RSV (131). Other examples include an inactivated measles vaccine (132,133), and possibly an ineffective caprine arthritis and encephalitis virus vaccine although the more severe arthritis following vaccination could have been due to antigen-antibody complex formation (134,135).

Lentivirus enhancing antibodies were first described for HIV infection in 1987 (110). Subsequent reports have identified two mechanisms for enhancement that function *in vitro*. The first involves antibody to HIV in combination with complement proteins (157,158) and requires cells that bear both CD4 and complement receptor type 2(CR2)(139). The second mechanism required antibody to HI Vand cells bearing Fc reports (140). Since the Fc mechanism generally has only a two fold enhancement versus > 100 fold for complement-mediated antibody dependent enhancement (C'-ADE), the present research focuses on the latter phenomenon.

For C'-ADE, it is known that the HIV envelope glycoproteins can activate complement and that antibody to HIV leads to increased fixation of complement component C3 on HIV or HIV-infected cells (141). This complement can bind HIV to CR2 and act to increase the amount of HIV in proximity to CD4+ cell surfaces resulting in a greater likelihood that the gp12- would interact with the CD4 receptor which mediates the entrance of HIV into the cell. Spear and his colleagues have directly shown that C'-ADE results in increased HI binding to target cells and an increased integrated proviral copy number (141).
Moreover, this group has shown that 30% of CD4 lymphocytes bear the CR2 receptor and that this CD4/CR2 lymphocyte is preferentially selected during the early phases of CD4 cell decline as a function of HIV infection (142).

With the production of huMAbs against the HIV-1 envelope glycoprotein, it is possible to separate virus neutralization from enhancement. It was shown that several huMAbs against the HIV-1 envelope glycoproteins could enhance HIV-1 infection but did not neutralize HIV-1 *in vitro* (143). The ability of the huMAbs to enhance infection was not determined by the ability of the huMAbs to activate complement nor by the IgG subclass of the huMAbs (143,144). These enhancing huMAbs have been mapped to linear domains in the HIV-1 gp41 transmembrane glycoprotein. Of six enhancing huMAb identified to date, five map to amino acid residues 579-613 (144,145), the primary immunodominant domain of gp41 (146-148). One of the six maps to another immunodominant domain (143,145). These results suggest that there are only a few enhancing domains in the envelope of HIV-1 and that these domains are conserved, immunodominant regions of the HIV-1 envelope. Recently, these observations have been extended to SIV by demonstrating that the TM protein regions homologous with the first and primary enhancing domain of HIV has similar capacity to induce the formation of enhancing antibodies (150). The data show *in vitro* that preimmunization with a synthetic peptide (aa 603-622) from SIV_{mac251} stimulated the production of antibodies which suppressed the beneficial effects of recombinant gp160 SIC vaccine and appeared to enhance SIV infection (150). There is further evidence from several Lentivirus vaccines that suggests that the humoral response to envelope glycoproteins may be detrimental to the host. For example, SIV envelope glycoprotein recombinant vaccines have, for the most part, failed to protect monkeys from subsequent virus challenge (151,152), while similar envelope-based recombinant vaccines for HIV have been largely ineffective in preventing HIV infection of chimpanzees (153- 155). In equine infectious anemia virus (EIAV), a baculovirus recombinant envelope glycoprotein vaccine apparently led to worse disease in horses subsequently challenged with EIAV (156). Recently, Gardner *et al*. (157) reported that passive immunization of rhesus macaques by serum from SIV-infected rhesus macaques led to an apparently enhanced course of disease with five of six such animals dying within 5 months of challenge. In that study, there was a direct correlation between failure of passive immunization and higher antibody levels against the ss 603-622 peptide by ELISA (158). These data differ from passive immunization experiments reported by Putkonen *et al.* (159) for SIV and in the feline immunodeficiency (FIV) model (160), although investigators have reported enhanced infections for FIV *in vitro* (161) and similar passive immunization failure for SIV_{mac} (162) *in vivo*. It is, therefore, prudent to consider the potential for enhancement in all vaccine preparations. However, HIV and SIV enhancement is relatively weak in comparison to Dengue (150).

### Induction of Mucosal Immune Responses In Vivo

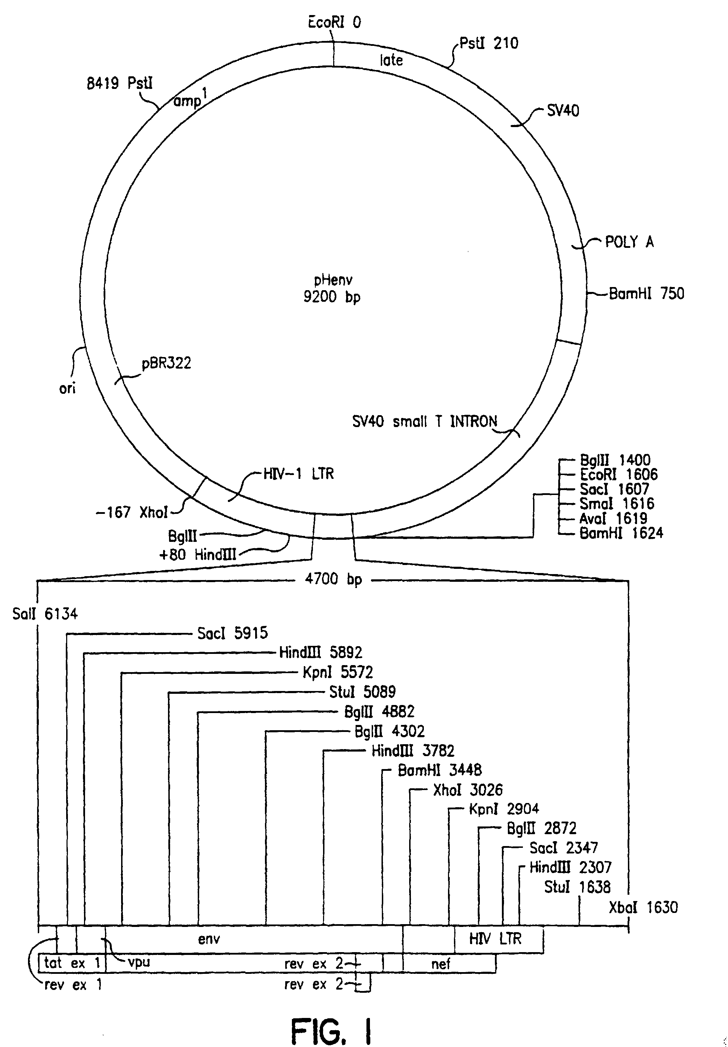

One of the transfection DNAs used as the genetic immunogen was pHenv, a 9600 bp pBR322-based plasmid containing the entire HIV-1 envelope genome, functional tat and rev transactivator sequences and corresponding LTR TAR and RRE sequences, obtained originally from the NIH AIDS Reference Program. Figure 1 details the circular plasmid map. This plasmid on transfection has been shown to efficiently express the env proteins of HIV- 1_{pNL4-3} resulting in extensive cell fusion with cells expressing CD4 (180). pACYC177 was used as an irrelevant DNA control immunogen. Plasmids were produced in *E. coli* JM109 grown in LB broth containing 50 µg/ml ampicillin and cells harvested at OD₆₀₀ₙₘ = 0.50. DNA was harvested from cell pellets using an SDS lysis procedure with purification on Magic Prep columns (Promega Corp.). Plasmid DNA was eluted at 70°C with TE buffer and precipitated at -70°C in 0.1 M Na acetate, pH 5.2. Purified plasmid DNA was examined by size in agarose electrophoresis gels for known restriction endonuclease hydrolysis sites. pHenv DNA was consistent with a 9600 bp DNA containing four HindIII sites producing 3708, 5818, 7293, and 9520 bp fragments. The 3940 bp control plasmid, pACYC177, contains one BamHI site and two StaI fragments of 965 bp and 2305 bp. Concentrations of DNAs were based on OD/|a\|a1(50µg, 260nm/|, 1cm) = 1.

### Summary of Results

Five- to six-week-old female Balb/c mice from Harlan industries were randomly sorted into groups of five mice each. The groups were arranged into three classes of DNA immunization plus irrelevant DNA immunogen and naive animal controls. Table 3 details the route of immunization, composition of immunogen, dose, number of immunizations, and total DNA dose exposure. Table 4 summarizes the antibody responses detected in the serum of mice as the result of transfection with DNA containing transcription competent HIV-1 env sequences. Naked DNA (100 µg) yielded HIV-specific serum IgG responses in 2 of 5 animals with a single IM exposure and 3 of 5 animals with 2 or 3 IM exposures. Naked DNA (10 µg) produced no HIV-specific immune responses with IM exposure. The highest average IgG titer occurred in the 10 µg per exposure group. DNA (10µg or 1µg) complexed with dioctadecylamidoglycylspermidine (DOGS) produced HIV-specific immune responses in 80% of IM treated animals after one, two, or three exposure with the highest average titer occurring in the group receiving three exposures. Nasal lavage exposure to 10 µg DNA complexed with DOGS produced systemic HIV-specific immune responses in 20% of mice with one exposure and 40% of mice exposed two or three times. Nasal lavages with 1 µg DNA complex produced specific IgG responses in 20% of mice after one or two exposures and 80% of mice following the third exposure with an average titer f 1:850.

**Table 3.**

| DNA immunization schedule in 5- to 6-week-old female Balb/c mice. Exposure to immunogens every three weeks. IM = intramuscular in divided doses (100µl) between the hamstring muscles bilaterally. NA = nasal lavage administered in small aliquots to 100 µl final volume. Naked DNA refers to pHenv-DNA in water. Dioctadecylamidoglycylspermidine (DOGS) was obtained from Promega Corporation (Transfectam® IBF-Sepracor, France) and complexed to DNA at a 5:1 molar cationic charge excess according to the manufacturer's directions. Complexes were prepared and used immediately prior to immunization. pACYC177 DNA was used as an irrelevant control DNA. | | | | | |
|---|---|---|---|---|---|
| Group # | Immunogen | Route of Immunization | Number of Immunizations | DNA/ Immunization | Total DNA |
| 1 | Naked DNA env | 1M | 1 | 100µg | 100µg |
| 2 | Naked DNA env | 1M | 2 | 100µg | 200µg |
| 3 | Naked DNA env | 1M | 3 | 100µg | 300µg |
| 4 | Naked DNA env | 1M | 1 | 10µg | 10µg |
| 5 | Naked DNA env | 1M | 2 | 10µg | 20µg |
| 6 | Naked DNA env | 1M | 3 | 10µg | 30µg |
| 7 | Naked DNA env | 1M | 1 | 1µg | 1µg |
| 8 | Naked DNA env | 1M | 2 | 1µg | 2µg |
| 9 | Naked DNA env | 1M | 3 | 1µg | 3µg |
| 10 | DOGS.DNAenv complex | 1M | 1 | 10µg | 10µg |
| 11 | DOGS:DNAenv complex | 1M | 2 | 10µg | 20µg |
| 12 | DOGS:DNAenv complex | 1M | 3 | 10µg | 30µg |
| 13 | DOGS:DNAenv complex | 1M | 1 | 1µg | 1µg |
| 14 | DOGS:DNAenv complex | 1M | 2 | 1µg | 2µg |
| 15 | DOGS:DNAenv complex | 1M | 3 | 1µg | 3µg |
| 16 | DOGS:DNAenv complex | NA | 1 | 10µg | 10µg |
| 17 | DOGS:DNAenv complex | NA | 2 | 10µg | 20µg |
| 18 | DOGS:DNAenv complex | NA | 3 | 10µg | 30µg |
| 19 | DOGS:DNAenv complex | NA | 1 | 1µg | 1µg |
| 20 | DOGS:DNAenv complex | NA | 2 | 1µg | 2µg |
| 21 | DOGS:DNAenv complex | NA | 3 | 1µg | 3µg |
| 22 | Naked control DNA | 1M | 3 | 100µg | 300µg |
| 23 | DOGS:control DNA complex | 1M | 3 | 10µg | 30µg |
| 24 | DOGS:control DNA complex | NA | 3 | 10µg | 30µg |
| 25 | None | - | 0 | 0 | 0 |

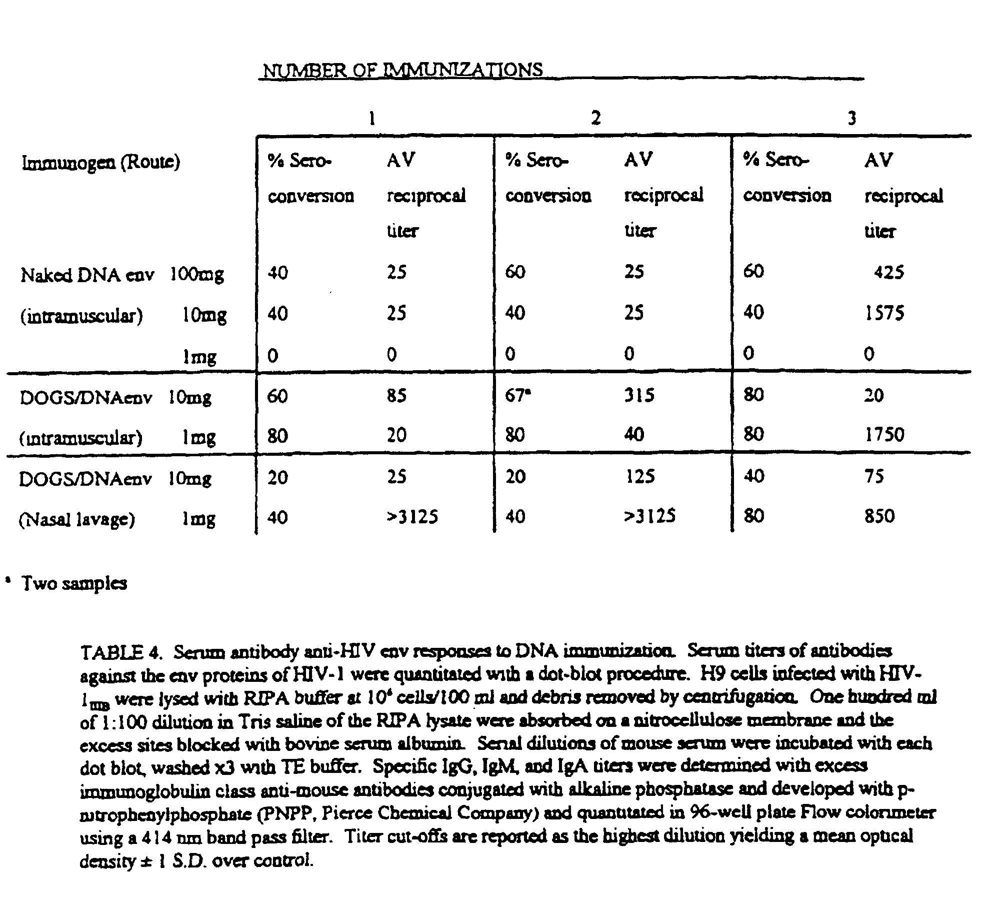

The Western blot immunoreactivity in seroconverted animals was determined. Western blots were prepared by SDS-PAGE of HIV-1_{IIIB} infected H9 cell lysates with transfer to nitrocellulose achieved with a four-day passive diffusion transfer. Albumin blocked strips were prepared from nitrocellulose sheets and incubated 1 hour with 200 µl of a 1:40 dilution of mouse serum. Detection was achieved with an alkaline phosphatase conjugated antimouse antibody and developed with 5-bromo-4-chloro-3'-indolyphospate p-toluidine/nitro-blue tetrazolium chloride (BCIP/NBT, Pierce Chemical Company). Human Ig (HIVIG) obtained from Fred Prince at the New York Blood Center was used as a human positive control with an anti-human alkaline phosphatase detection system. Mouse antisera generated against the terminal twelve amino acids of gp120 was used as a mouse positive control. This antiserum was produced by F-MOC peptide solid phase synthesis, purification of the peptide by HPLC on a C18 column using a TFA/H₂O gradient, conjugation to KLH, and administration to mice by multiple intradermal injections using Freund's adjuvant. Western blot analysis indicated that the bulk of the humoral immune response was directed at gp41 demonstrating clear anti HIVenv systemic response to direct mucosal genetic immunizations.

Immunohistochemistry of lung and colon in animals from the nasal lavage group indicated that a specific response to HIV-env was present on bronchiolar and colonic mucosal surfaces. An IGA-specific alkaline phosphatase decoration of lung tissue in an animal immunized with a nasal lavage of DOGS/HIVenv-DNA was observed. Control animal consistently revealed no labeling of IgA on mucosal surfaces. An anti-HIVenv reactivity of a bronchiolar epithelium in a lung from a mouse immunized by the nasal lavage delivery of DOGS/HIVenv-DNA was demonstrated. Control animals demonstrated no bronchiolar reactivity towards HIVenv antigens. A fluorescent antibody decoration of coionic mucosa from an immunization via nasal lavage was observed. This visualization of IgA responses following genetic mucosal immunization and the finding of HIV envelope proteins from H9/IIIB infected cells represents a specific secretory IgA response to mucosal genetic immunization. This is the first demonstration of a mucosal immune response induced by a genetic immunogen.

Although there is extensive experience with the measurement of human neutralization and enhancing antibodies, initial efforts with Balb/c mouse serum revealed an HIV inhibitory factor which has been inactivated with heat at 56°C for 1/2 hour. Further neutralization assays using heat inactivated serum will be rigorously standardized using heat inactivated Balb/c serum from mice immunized with HIV-_{IIIB} as a positive control. Mice are currently being immunized with inactivated HIV-1 _{IIIB}. Additionally, CTL responses to HIVenv genetic immunogens can be quantitated.

Histochemical staining activities in mice immunized with DOGS/HIVenv DNA nasal lavage was performed. Five-micron frozen sections were prepared from snap frozen (liquid nitrogen) lung and colon of nasal lavage immunized mice using a refrigerated microtome and adhered to standard silinized glass slides. To demonstrate mucosal antibodies specific for HIVenv determinants, each section was incubated for 30 min to a 1:100 dilution of H9/IIIB cell lysate. The sections were extensively washed with T buffer and incubated with 100 µl of a 1:100 dilution of HIVIG. Binding of human Ig was detected after extensive washing in TE buffer with a goat anti-human IgG antiserum conjugated with alkaline phosphatase and developed with BCIP/NBT detection of mouse anti-HIV mucosal antibodies in lung and vaginal sections and goat anti-human IgG antiserum conjugated with fluorescein for detection of anti-HIV mucosal antibodies in jejunum and colon. Mucosal IgA antibodies were visualized in lung frozen sections using a goat anti-mouse IgA coupled with alkaline phosphatase or fluorescein.

The binding of DOGS to DNA has been studied by molecular modeling using the DREIDING II force field in a Biograf software package (Molecular Simulations, Inc.) on a Silicon Graphics RISC-based computer. After molecular dynamics the molecule is seen to bind to the major groove of DNA. Two of the four positive charges on the polar end of DOGS plus the peptide amide hydrogen symmetrically coordinate with a single phosphate on DNA. One hydrophobic arm extends from the phosphate while the second extends in the opposite direction along the major groove for several methylene groups then bends toward the first arm. This conformation places a sequence of 4-5 methylene groups exposed to solvent per phosphate. One of the DOGS lipophilic chains extends into solvent. With a molar charge ratio of 5:1::DOGS/DNA, all phosphate groups will be ionically complexed with a hydrophobic shell covering the entire DNA molecule. This explains the stability of the complex and its affinity for the lipid plasma membrane. It is possible that a structure of this type could survive passage through the acidic environment of the stomach if the DOGS/DNA charge complex is relatively inaccessible to hydrogen ions.

### 2. Vector Design and Synthesis

The vector used for the pilot genetic immunization study, pHenv, is a 9600 bp plasmid containing SV40 promoters. The HIV envelope sequences, however, are contained within LTRs with fully functional tat and rev genes plus the RNA receptor sites TAR and RRE, respectively. Presumably, the expression of gp160 is under LTR promoter control. It can now be determined whether this expression system is more advantageous for expressing the env proteins and for inducing functional immune responses than the use of other promoters (CMV) in constructs lacking function rev. Not only gp160 but gp120 and gp41 can be expressed separately or in combination.

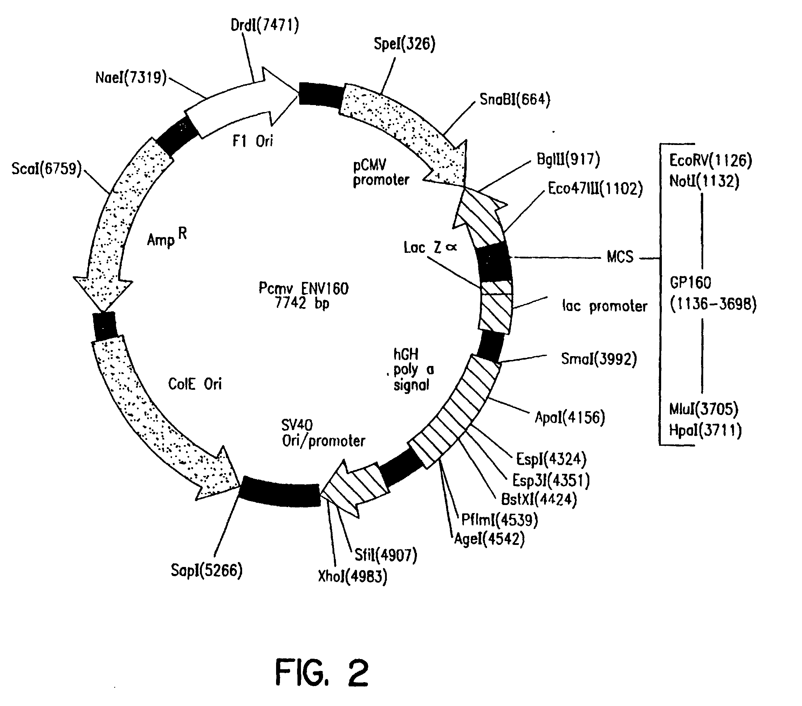

One possible problem is the extensive secondary structure of the RRE which has caused difficulties in the initial site-directed mutagenesis efforts on the primary enhancing domain of gp41. We have, therefore, introduced silent mutations (*i.e.*, no alteration amino acid sequence of gp41) designed to abolish the secondary structure of the RRE. We have produced two such mutations. RRE-3C is designed to disrupt the binding site for Rev while RRE-4C will more extensively disrupt the entire RRE. Figure 2 illustrates the RRE and the mutagenized sites. pHenv was cut with SalI and EcoRI and the 4700 bp gp160 sequence isolated from agarose following electrophoresis. This sequence was cloned into the pAlter MCS, subjected to site-directed mutagenesis, on the RRE, ampicillin (repair), and tetracycline (sensitivity) sites and selected with ampicillin. Mutagenized RRE yield the correct size and restriction sites following antibiotic selection. Sequence analysis confirmed the authenticity of the RRE mutations. Surprisingly, RRE under a CMV promoter and lacking a functional rev protein yielded better immune responses than a RRE negative construct (Table 6). Similarly, RRE *in vitro* is required for maximal translation of gp160. Thus, the presence of an intact ARE is a desirable feature in the construction of a genetic vaccine for the envelope of HIV even in the absence of rev.

### 3. Molecular Modeling of the HIV Envelope Proteins

Although the primary sequence of the HIV env-proteins are hypervariable, certain structural features remain constant among all isolates. Figure 2 is a representation of the structural data for gp120 reported by Leonard et al. (174) in which disulfide pairings were identified by classical chemical methods and generic glycosylation patterns (sialated vs non-sialated) were assigned by enzymatic methods. Included in Figure 2 is the single disulfide present in gp41 and its N-linked glycosylation sites of unknown structures. These structural relationships are combined with the neutralizing and enhancing antibody binding domains reviewed by Robinson and Mitchell (178). There are nine disulfide bonds and one free sulfhydryl in gp120. The positions of the cysteine residues in gp120 is highly conserved in all isolates with the exception of the Z3 isolate in which an additional two cysteines are present in the fourth hypervariable domain. This strongly suggests that the disulfide pairing in III_{B} is maintained among all isolates. Using the Dreiding II generic force field for molecular simulations, Gabriel and Mitchell (172) have generated molecular simulations for a truncated gp120 which agree with all known data concerning gp120 glycosylation, antigenic structure and gp120/CD4 binding interactions. Docking inhibition studies with known gp120/CD4 binding inhibitors have recently been completed that provides further credence to the model (179). Similar modeling studies with gp41 in which the Cys-Cys loop of gp41 has been docked to the C-terminal concavity on gp120 in agreement with the theoretical predictions of Moore *et al*. (149) having been conducted. Thus, there is a reliable model for the relationships between the major antigenic sites on gp120 and N-linked oligosaccharides, gp120/gp41 interaction, and gp20 docked to CD4. The V3 loop of gp120 is solvent accessible on one face although a portion is obscured by carbohydrate. The second conserved domain is accessible from one side only. The CD4-binding domain is completely obscured by carbohydrate on one face but is easily accessible on the opposite face. The present ability to provide a model structure in which the effects of sequence variation in the genetic immunogens can be predicted is a valuable tool that will aid us in their design and analysis of those biological responses that are dependent on structural changes in the protein immunogen.

### 3. Significance to the HIV Vaccine Program

Specific anti-HIV env responses have been generated in Balb/c mice by facilitated genetic immunization of mucosa. Mice are currently being immunizing against HIV-1_{IIIB} in order to obtain a mouse polyclonal control HIV neutralizing serum obtained by conventional methods. The quantitation of functional humoral responses towards HIV in humans can be done as taught herein and no difficulty is anticipated. Since genetic immunization has been shown to generate CTL responses, two unique cell lines have been obtained from Viagene Inc. (San Diego, CA) that can be used to evaluate cytotoxic lymphocyte (CTL) responses in Balb/c mice as a function of immunization (182). The Hu/D^{d} line is a CD4 expressing HeLa derivative that carries and expresses the D^{d} MHC locus of the Balb/c mouse. This line can be infected with a wide variety of established and primary HIV isolates to be used as a target for Balb/c CTLs. The second cell line is a Balb/c fibroblast that has been permanently transfected with HIVenv (IIIB) sequences, which are expressed on the cell surface. This cell provides another suitable target for CTL analysis in Balb/c mice as a function of immunization against HIV.

### 4. Method Design and Protocols

### A. General Design

The initial evaluation used pHenv as the common vector in the direct comparison of mucosal and systemic responses to genetic immunization. Balb/c mice have been evaluated for serum titers of Igs, Western blot and radioimmunoprecipitation assay (RIPA) specificities of immune humoral responses, sIgA titers in parotid secretions, and direct visualization of mucosal antibodies specific for HIV. Neutralizing titer of serum and parotid antibodies and spleen CTL activity against ⁵¹Cr labeled against target Bcenv and Hu/D^{d}/HIV can also be determined routinely. The ability of DOGS/DNAenv complexes to induce a common mucosal response will be evaluated with nasal lavages, colonic exposure, vaginal exposure, and gastric delivered formulations. In each case a dose response analysis will be done using 10 µg DNA as the highest total DNA single dose exposure. Similarly an evaluation will be done of the responses as a function of one, two, or three genetic immunization schedules allowing 2 week and/or 3 month intervals between responses. The inclusion of the vitamin in the bolistic DNA/Au formulations can be evaluated in order to establish whether a single formulation is possible. The stability of the formulation can be routinely establish under various physical conditions.

While evaluating the various routes and modes of genetic immunization outlined above, a variety of DNA constructs can be developed for use in genetic mucosal immunization. The effect of various eukaryote promoters on immunogen expression is routinely examined. The most effective eukaryotic promoter examined to date for genetic immunization is the CMV promoter for HBV subunit expression (18) although other more effective promoters may be found. The thymidine kinase promoter for herpes simplex virus (HSV) is examined using pTKb (GenBank accession # U02438), the SV40 early promoter using pSVb (GenBank accession # U02435), the CMV immediate early gene promoter using pCMV-Lic (Contains CMV promoter/enhancer LIC cloning site, HGH polyadenylation site, and SV40 early promoter) or pCMvb (GenBank accession #U02451), and the adenovirus major late promoter (GenBank accession # U02442). Using gp160 lacking the LTRs, it can be evaluated which vector provides maximal expression in a variety of eukaryote cell lines, such as HeLa and SG181 (a human fibroblast) and human H9 as well as primary mouse lung, intestinal, and skin explant organ cultures. Those promoters providing the best consistent expression will be used in the subsequent vector constructs.

Following identification of the best promoter(s) for surface expression in target eukaryote cells, vector constructs will be prepare which are designed to identify the best signal peptide sequences (*i.e*., the HIV signal sequence versus TPA signal peptide, for example), expression from a vector carrying the RRE secondary structure, gp160 versus gp120 and/or gp41 containing the gp160 membrane anchor domain, and gp160 in which the gp160 cleavage site has been eliminated. Following identification of the best constructs for genetic immunization a vector will be constructed with HIVenv sequences plus p17 since an N-terminal neutralization site has been identified in this HIV gag protein. Using the teaching herein, the best plasmid vector construct can be determined in order to maximize both the quality and quantity following genetic immunization.

Genetic immunization offers the best opportunity for generating multiple responses to the various hypervariable forms of the Principle Neutralizing Domain of HIV (*i.e*., V3 loop). V3 loop mutations on a pNL4-3 DNA envelope sequence can be generated that reflect the major macrophagetrophic and lymphotrophic variants of clade B viruses as well as those limited V3 loop sequences recognized to be the infectious variant from a multiplicity of potential infectious variants of the infecting donor (171). For each V3 loop variant, the effect on gp120/gp41 conformation can be examined by molecular modeling in order to anticipate alterations on group-specific conformational epitopes. Also, the response to DNAenv cocktails containing multiple V3 loop species can be examined using the methods taught herein.

### Detailed Protocols

### 1) Facilitated DNA env mucosal immunization

Diotadecylamidoglycylspermine (DOGS) obtained from Promega as Transfectam® will be solubilized in 100% ethanol and complexed to DNA in H₂O at a 5:1 molar cationic charge excess and diluted in Tris-saline to the immunization dose based on DNA concentration and administered immediately following formulation. One hundred µl will be administered as a nasal lavage, gastric bolus, or colonic bath or 25 µl deposited intravaginally to anesthetized (ketamine/xylazine) female Balb/c mice of six weeks or six months age. All animals will be randomized into groups of five. Each animal will be euthanized by exsanguination under ketamine/xylazine anesthesia three weeks after the final immunization. Whole blood will be collected by abdominal aorta catheterization (#25 pediatric cut-down set). The spleen will be collected and teased for white pulp and PBMCs isolated on Hypaque-Ficol. Lungs, colon, small intestine, and vagina will be collected and snap frozen in liquid nitrogen for subsequent frozen section processing.

### 2) Vector Constructs

a. Vector constructs use a plasmid vector containing a CMV promoter, a multicloning site (MCS) and a poly-A signal site. HIVenv DNA sequences are amplified by PCR with unique overhang restriction sites for insertion into the parent plasmid MCS. For this purpose, the NotI/MluI sites were used for the construction of native and mutant DNA immunogens.
b. Site-directed mutagenesis:
   The P-Alter® method was routinely used for the introduction of mutations at specific sites. A SalI/EchoRI agarose purified restriction fragment of pHenv containing the HIV_{NL4-3} envelope sequence was cloned into the p-Alter vector. This vector contains a mutant ampicillin resistance gene and a tetracycline resistance gene for selection during multiple rounds of additive mutagenesis. JM109 *E. coli* transformed with p-Alter_{HIVenv} are induced to produce single strand (ss) DNA using helper phage DNA. Three mutational primers are hybridized at room temperature to the ssDNA (ampicillin resistance repair primer, tetracycline resistance inactivation primer, and a mutational primer of the gene under analysis). The hybridized DNA is filled in with T7 polymerase and mutant repair *e. coli* used for transformation and mutagenized plasmid recovery on antibiotic selection plates. This method has provided the highest yield of desired sequence verified mutations of the various methods tested. The critical factors concern the purity of phage DNA and the use of MutS-Blue *E. coli* lacking *all* DNA repair systems.

### 4) Systemic Antibody Analysis

a. Ig Titers. Serum titers of antibodies against the env proteins of HIV-1 are quantitated with a dot-blot procedure. H9 cells infected with HIV-1_{IIIB} are lysed with RIPA lysis buffer (0.05 M Tris-HCl, pH 7.2, 0.15 M NaCl, 0.1% SDS, 1% Triton X-100, 1% deoxycholate, 1 mM phenyl methyl sulfonyl fluoride) at 10⁶ cells/100 µl and debris removed by centrifugation. One hundred µl of 1:100 dilution in Tris saline of the RIPA lysate are absorbed on a nitrocellulose membrane and the excess sites blocked with bovine serum albumin. Serial dilutions of mouse serum are incubated with each dot blot, washed X3 with TE buffer. Total IG or specific IgG, IgM, and IgA titers are determined with excess anti-mouse Ig antibody immunoglobulin class anti-mouse antibodies conjugated with alkaline phosphatase and developed with p-nitrophenylphosphate (PNPP, Pierce Chemical Company) and quantitated in 96-well plate Flow colorimeter using a 414 nm band pass filter. An optical density scanner allows the performance of OD scans directly in dot blots. Titer cut-offs are reported as the highest dilution yielding a mean optical density ± 1.S.D. over control.
b. Western blot. Western blots are prepared by SDS-PAGE of HIV-1_{IIIB} infected H9 cell lysates with transfer to nitrocellulose achieved with a four-day passive diffusion transfer. Albumin blocked strips are prepared from nitrocellulose sheets and incubated 1 hour with 200 µl of a 1:40 dilution of mouse serum. Detection is achieved with an alkaline phosphatase conjugated anti-mouse antibody and developed with 5-bromo-4-chloro-3'-indolyphosphate p-toluidine/nitro-blue tetrazolium chloride (BCIP/NBT, Pierce Chemical Company). HIVIG obtained from Fred Prince at the New York Blood Center is used as a positive control with an anti-human alkaline phosphatase detection system.
c. Radioimmunoprecipitation analysis (RIPA). H9/IIIB cells are labeled with ³⁵S-cysteine in a cysteine-free medium for 4 hours at 1 mCi/ml containing 1 x 10⁶ cells. The cells are washed X3 in PBS lysed in RIPA buffer (see 4a above). Attempts were made to achieve 20 x 10⁶ cpm with 2 x 10⁵ cpm/µl. Sera to be tested are incubated with 100 µl of a diluted Protein G-sepharose (Pierce) for 1 hour at 4°C. Lysate is added at an equivalence of 0.5 to 1 x 10⁶ cells. The serum antibodies and lysate antigens are incubated overnight at 4°C, washed in RIPA wash buffer (*i.e*., RIPA lysis buffer minus deoxycholate and phenyl methylsulfonyl fluoride). The immune complex-Protein G beads are centrifuged at 1000 g, washed x3 with 4 ml RIPA wash buffer, denatured at 100°C for 2 minutes and run on SDS-PAGE in 10% resolving gels. After electrophoresis the gel is fixed in 30% methanol, 10% acetic acid, 60 ddH₂O or equivalent and radioactive bands visualized with a Molecular Dynamics PhosphoImager.
d. Neutralization Assays.
   (i) standard microtiter neutralization assay. Neutralizing antibody activities will be measured in microtiter infection assays as originally described from this lb (183). Briefly, heat-inactivated (60°C, 30 min.) serum samples will be two-fold serially diluted in triplicate into RPMI 1640 growth medium containing 12% FCS. Virus will be added (5-10 x 10⁵ infectious units) and incubated at 37°C for one hour. Next, 2-5 x 10⁵ MT-2 cells in 100 µl of growth media will be added to each well and the plates incubated for 2-3 days at 37°C in 5% CO₂/95% air. Cells will be monitored by phase contrast microscopy for syncytia formation and assayed when virus control wells (no mouse serum) show extensive cytopathic effect. This usually is at 3 1/2 days when MOI≥1 is used. Cells are transferred to poly-L-lysine coated plates and incubated with Fainter's neutral red dye for 1 hr. Adherent cells are washed with phosphate-buffered saline (PBS) and vital dye liberated with acid alcohol. Plates will be analyzed on a Flow Titertek Microcolorimeter at 540 nm for viable cells. Viability will be determined relative to the cell control wells (n = 4). Neutralizing titer is defined as the highest dilution yielding ≥50% cell viability compared to cell control.
   (ii) Primary isolate neutralization. The gold standard for neutralization is the ability to neutralize the ability of a panel of primary isolates to infect human PBMC. The latter are freshly isolated on Hypaque-Ficol. 5 x 10⁶ cells in 10 µl of undiluted primary isolate HIV (*i.e*., always propagated on PBMCs) are incubated in triplicate in serial 5 fold dilutions of mouse serum for 1 hour at 4°C and then added to 1 ml RPMI/12% FCS containing biological derived IL2. Supernatants at 7 days are assayed for RT and/or P24 levels versus control cultures. The highest diluticn to yield ≥ 50% inhibition is reported as the neutralization titer.

### 5) Mucosal Antibody Analysis

a. Parotid secretion IgA/IgG titers: Titers will be monitored weekly for short term immunization schedules and monthly on long term schedules. Parotid secretion in anesthetized (IM ketamine/xylazine) Balb/c mice will be induced with pilocarpine (20 µg/mouse) and saliva collected on specified days with a Pasteur pipette. Analysis of 2 fold serial dilutions will be determined by a dot blot procedure described under 4a above. Detection of specific responses to various V3 loop expression immunogens, parotid secretions will be titered using V3 loop peptides synthesized on the Miligen 9050 peptide synthesizer by the F-moc method. One µg of synthetic peptide in 100 µl of coating buffer (0.1 M bicarbonate buffer [ph 9.6]) are added to each well of Immulon 2 microtiter plates and incubated at 37°C for 2h. The wells are next washed three times in phosphate-buffered saline (PBS) containing 0.05% Tween 20, and then serially diluted (2 fold) saliva is added to each of triplicate wells. After incubation for 2 h at 37°C, the wells were washed three times with washing buffer. Next, goat anti-mouse immunoglobulin A or G (heavy- and light-chain specific) coupled to horseradish peroxidase is added at a dilution of 1:1,000 and incubated for another hour at 37°C. After the wells are washed five times with PBS containing 0.05% Tween 20, 2,2'-azino-bis(3-ethylbenzthiazoline sulfonate) (ABTS) is added as the substrate and incubated for 30 min at room temperature. The optical density (OD) of each well is read in an enzyme-linked immunosorbent assay (ELISA) reader at 410 nm. For more detailed descriptions of the procedures and analysis please refer to references 143-145.
b. Immunocytochemistry: Five-micron frozen sections are prepared from snap frozen (liquid nitrogen) lung, colon, jejunal and vaginal tissues of genetically immunized mice using a refrigerated microtome and adhered to standard silinized glass slides. To demonstrate mucosal antibodies specific for HIVenv determinants, each section is incubated for 30 min in a 1:100 dilution of H9/IIIB cell lysate (1:100::RIPA:Tris saline). The sections are extensively washed with TE buffer and incubated with 100 µl of a 1:100 dilution of HIVIG. Binding of human Ig (HIVIG) is detected after extensive washing in TE buffer with a goat anti-human IgG antiserum conjugated with alkaline phosphate and enveloped with BCIP/NBT detection of mouse anti-HIV mucosal antibodies in lung and vaginal sections and goat anti-human IgG antiserum conjugated with fluorescein for detection of anti-HIV mucosal antibodies in jejunum and colon. Mucosal IgA and IgG antibodies are visualized in frozen sections using a goat anti-mouse IgA coupled with alkaline phosphatase or fluorescein.

### 6) Analysis of Cytotoxic Lymphocyte Activity against HIV Expressing Targets (CTLenv)

CTLenv will be quantitated using the Hu/D^{d} cell line infected with primary HIV isolates or the murine Bcenv line (HIV expressing) as described earlier under summary of results. Uninfected Hu/D^{d} and BCgal (b galactosidase expressing) will be used as controls, respectively. Target cells will be intracellularly loaded with ⁵¹Cr by incubation for 45 min under 5% CO₂ 1.5 x 10⁶ cells in RPMI 1640 with 150 µl Na₂⁵¹CrO₄ in PBS (1 mCi/ml, specific activity 400- 1200 Ci of Cr per gram from DuPont/NEN). Labeled cells are washed in cold RPMI/10% FCS x3 and kept on ice for cytotoxicity assay. Mouse spleen mononuclear cells isolated by Hypaque-Ficol are added to target cells (10⁴ cells in 100 µl RPMI 1640/10% FCS) at effector:target ratios of 100:1, 50:1, 25:1, and 12.5:1, incubated at 37°C under 5% CO₂ for 4 hours, centrifuged, and 100 µl counted in a Gamma counter. Control target cells are lysed with 5% Triton X-100 to obtain maximal release values and cytotoxicity calculated by % cytotoxicity - \|f(exptl release - spontaneous release, maximal release - spontaneous release ) x 100.

### 7) Analysis of the Persistence of Transfected DNAenv in Tissues

Primary transfection site tissues will be harvested as a function of time following transfection and aliquots lysed in 1% Triton X-100, 10 mM Tris, pH 7.0, and 1 mM EDTA, centrifuged at 1000 x g to remove insoluble debris, and the supernatant removed and heated to 100°C for 5 minutes. Analysis for DNA env will use PCR amplification of the V3-V5 regions using EDS (5'-ATGGGATCAAAGCCTAAAGCCATGTG) and ED12 (5'-AGTGCTTCCTGCTGCTCCCAAGAACCCAAG) primers which yields a 1200 bp DNA product corresponding to - bp 6160-7358. Standard conditions for this gene product in a 50 µl volume is 35 cycles with 1 second ramp times between steps of 94°C for 60 s, 55°C for 60 s and 72°C for 120 s with cycling initiated following a 5-min incubation at 95°C and wax bead "hot start." The PCR reaction used 0.2 µM of each primer in 50 mM KCl, 10 mM Tris-HCl (pH 8.3), 200 µM of each dNTP, 2.5 U Taq DNA polymerase and 1.5mM MgCl. Two to ten ul of the cell lysate is used as template. Amplified DNA is separated and identified by electrophoresis in 1.2% agarose or 6% polyacrylamide gels run in THE buffer (88 mM tris-borate, 89 mM boric acid, 2 mM EDTA) at 120 volts for 1 hr. DNA bands are identified by ethidium bromide straining and UV light detection. Primer specificity is verified by using pNL4-3 plasmid-derived DNA and total genomic obtained from ACH-2 cells (positive control).

### 8) In situ Analysis of DNAenv Transfected Cell Types

*In situ* hybridization of a PCR amplified DNA using suitable probe of high specificity will allow the detection of transfected DNAenv in a normal cellular architecture that would otherwise be undetectable. Biopsy specimens from the transfected tissues are fixed for 1 hour in a non-crosslinking, water soluble fixative (Strekk Tissue Fixative (STF)], embedded in paraffin tissue blocks, sections mounted on polylysine coated class slides and processed for routine H&E histology. In order to perform PCR amplification, 4 µm sections containing three sections per slice are deparaffinized by successive washes in xylene and progressively diluted alcohol solutions. Deparaffinized slides are subjected to proteinase K permeabilization of the plasma membranes (10 µg/ml for 20 min at RT). Each membrane permeabilized slide is then placed on the hot stage (5°> primer melt temperatures) of the Perkin-Elmer In Situ PCR slide Prep apparatus. Two sections serve as controls (*i.e*., one lacking primers as negative control and a second using a housekeeping gene amplification such as F-actin as positive control for membrane permeabilization). Thirty-five µl of PCR mix containing the appropriate ions and pH found optimal for solution PCR (*i.e*., MgCl₂, KCl, in 10 mM Tris-HCl) and 7.5 units of Ampli-Taq DNA polymerase) plus the primers described above for solution PCR but containing a 5'-biotin (prepared on the DNA Cyclone) plus primers for the housekeeping control. Each section is sealed with a disposable plastic chamber and external metal clamp that serves as a heat sink. Each prepared slide is transferred in succession to the In Situ PCR Cycler held at the temperature Slide Prep apparatus and which has a capacity of ten slides. Temperature cycling times are those previously established using solution PCR. This procedure provides a hot start to minimize non-specific primer binding and polymerase extension during the procedure setup. Following in situ amplification of specific DNA sequences, detection is provided by streptavidin conjugated to alkaline phosphatase to detect specific sequence of the amplified DNA containing 5'-biotin. The water soluble substrate (nitrobluetetrazolium and 5-bormo-4-chloro-3'-indoylphosphate p toluidine) is precipitated at the site of enzyme catalyzed substrate hydrolysis forming a blue stain of transfected cells. This technology can utilize the Perkin-Elmer In Situ PCR equipment.

### HORMONAL IMMUNOMODULATED PERIPHERAL GENETIC IMMUNIZATION (HIPGV)

### DOGS/Vitamin D3 facilitated transfection.

### Summary of Results

Except as otherwise indicated, the protocols for the cationic lipid/DNA methods described above are used in the cationic lipid/DNA/Vitamin D3 methods described in this example.

The present experiments show that 1,25(OH)₂D3 serves as a molecular switch to add mucosal immune responses to the systemic response. More importantly, evidence that 1,25(OH)₂D3 added to a facilitated genetic immunogen induces both systemic and mucosal responses is provided. Using the pCMV-env160 containing an intact RRE, humoral responses were induced by facilitated transfection of mouse muscle in which 1,25(OH)₂D3 was included in the inoculum. Four of four mice responded at 2 weeks with significant titers of IgG, IgM, and IgA against gp160 (Table 5). sIgA titers were further examined following facilitated genetic immunization using 10 µg and 1 µg DNA. This was further analyzed as a function of the presence or absence of the RRE. All inoculations also included 1 µg 1,25(OH)₂D3. One animal in the 10 µg DNA dose and two animals in the 1 µg DNA dose with mutated RRE developed significant IgA titers in the parotid secretions at two weeks (i.e., 38% total response rate). In contrast all animals receiving 10 µg or 1 µg DNA doses in which the RRE was intact developed IgA titers against gp160 (Table 5). Moreover if the activated vitamin D3 is not present, no significant sIgA is observed in parotid secretions despite good systemic responses to genetic immunization (Table 7). Six of seven in the vitamin D3 group exceeded the detection limit of 1:1250. Thus, a 100% response rate for IgA in parotid secretions (n = 7) was observed, which is dependent on the presence of 1,25(OH)₂D3. IgA immunoprecipitation analysis of parotid secretions (10 µl) from an immunized animal reveals a relatively strong gp41 band and traces of gp160 and gp120 when compared to a HIVIG control. Complicating the interpretation of this RIPA analysis are minor bands in control parotid and U937 extract mimics antibody and seronegative mouse serum. A minor 35S component in U937/IIIB cells could be binding to protein A in the absence of specific antibody/env protein. Other HIV producer cell lines can be examined to try to eliminate this artifact.

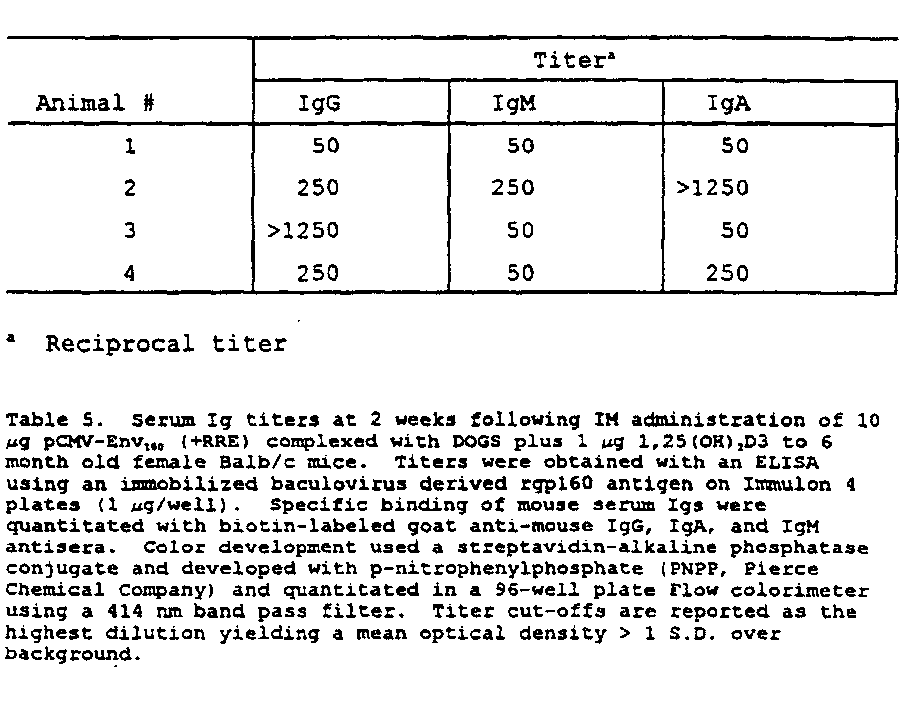

**Table 6.**

| Parotid IgA anti-HIV responses to DNA immunizations in 6 month old Balb/c mice. The DMA immunogen used a CMV promoter with a downstream gp160 insert expressing a native mRNA (+RRE) or a mutated mRNA lacking the RRE secondary structure (-RRE). All DNA immunogens were complexed with DOGS and contained 1µg 1,25(OH)₂D3. IgA titers were obtained as described in Table 5. No IgA anti-gp160 responses (i.e., <10) were detected in naive animals. | | | | | | | | |
|---|---|---|---|---|---|---|---|---|
| 1,25(OH)₂D3(1 µg) | | | | | | | | |
| pCMV-env160 (-RRE) | | | pCMV-env160 (+RRE) | | | pCMV-env160 (+RRE) | | |
| Animal No. | Dose | Titer^{a} | Animal No. | Dose | Titer^{a} | Animal No. | Dose | Titer^{a} |
| A1 | 10µg | <10 | C1 | 10µg | >1250 | C | 10µg | <10 |
| A2 | 10µg | <10 | C2 | 10µg | 250 | CL1 | 10µg | <10 |
| A3 | 10µg | >1250 | C3 | 10µg | >1250 | CR1 | 10µg | <10 |
| A4 | 10µg | <10 | C4 | 10µg | >1250 | CR2 | 10µg | 10 |
| B1 | 1µg | <10 | D1 | 1µg | >1250 | D | 1µg | 10 |
| B2 | 1µg | 10 | D2 | 1µg | >1250 | DL1 | 1µg | 10 |
| B3 | 1µg | >1250 | D3 | 1µg | NT^{b} | DL2 | 1µg | 10 |
| D4 | 1µg | 250 | D4 | 1µg | >1250 | DR1 | 1µg | 10 |

| | | | | | | | | |
|---|---|---|---|---|---|---|---|---|
| ^{a} Reciprocal titer. | | | | | | | | |
| ^{b}NT = not tested (anesthetic death). | | | | | | | | |

Using a baculovirus derived rpg160 labeled with biotin as a specific tissue probe for gp160 binding sites, tissues were examined for qualitative evidence of gp160 binding at mucosal surfaces. Organs from mice immunized with CMV-Env160 (+RRE) and CMV-Env160 (-RRE) by muscle transfection of DNA/DOGS complexes in the presence of 1 µg 1,25(OH)₂D3 were fixed in buffered formalin, embedded in paraffin, thin-sectioned to glass slides, and deparaffinized in graded alcohol. Sections were exposed for 1/2 hr to rgp160-biotin, washed x3 in Tris-HCl, pH 7.2, incubated with streptavidin-β-galactosidase, washed x3, and developed with X Gal (5-bromo-3 indoyl-β)-galactopyranoside at pH 7.6 (mammalian β-galactosidase is inactive at this pH) for 1/2 hr. Slides were counterstained with Fast Red.

Histochemical evidence of mucosal binding sites were observed in lung, large and small intestine, and uterus/vagina which are interpreted as sIgA based on location. Extensive marking is seen on bronchial mucosa as well as alveoli. gp160 binding extends over villi of the jejunum down to crypts. In colon, the surface is marked for gp160 binding. gp160 binding is present on both uterine and vaginal mucosa. Controls consisted of adjacent tissue sections in which the gp160-biotin probe was omitted from the incubation medium and naive animals in which all components were present. The only non-specific tissue marking detected is on serosal surfaces of naive mouse controls.

In addition tissue was examined at two weeks postimmunization. There is an inflammatory response at an immunization site with intense mononuclear cell influx in an area of necrotic muscle in which the majority of mononuclear cells are marked with the gp160-biotin probe.

In addition to DOGS, we have examined the utility of a second cationic lipid (TEDBI) to facilitate *in vivo* DNA immunization. Table 7 illustrates systemic Ig responses at charge ratios of 3:1 and 4:1 (cationic lipid:DNA). This is the first example of the ability of this cationic lipid to facilitate DNA transfection *in vivo*. Table 8 shows Ig responses to doses of TEDBI complexed DNA.

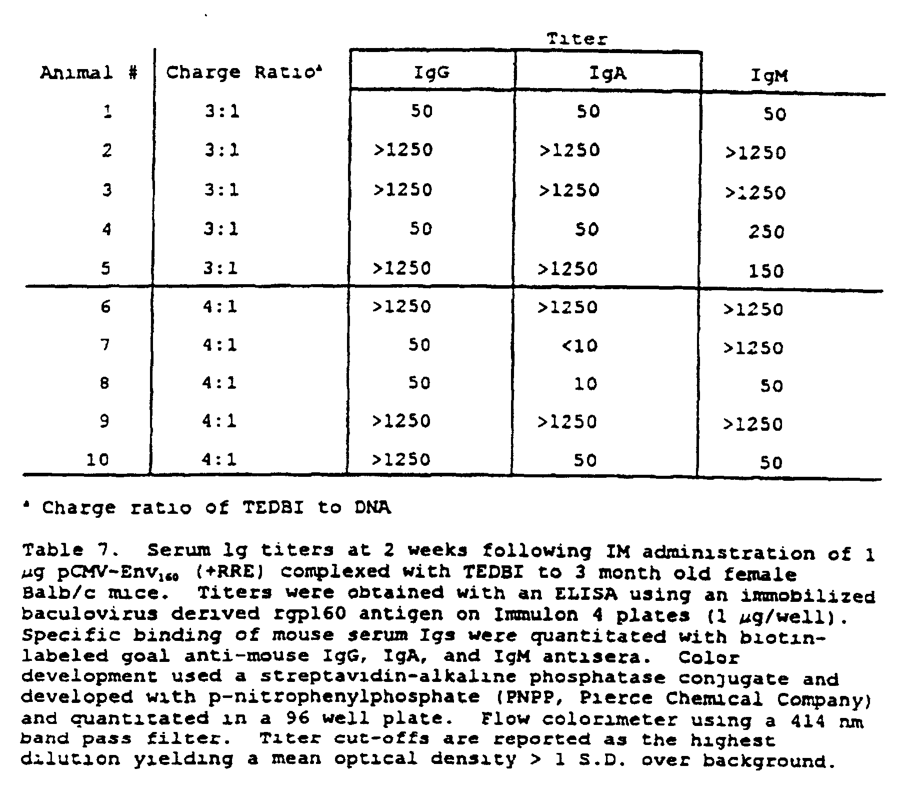

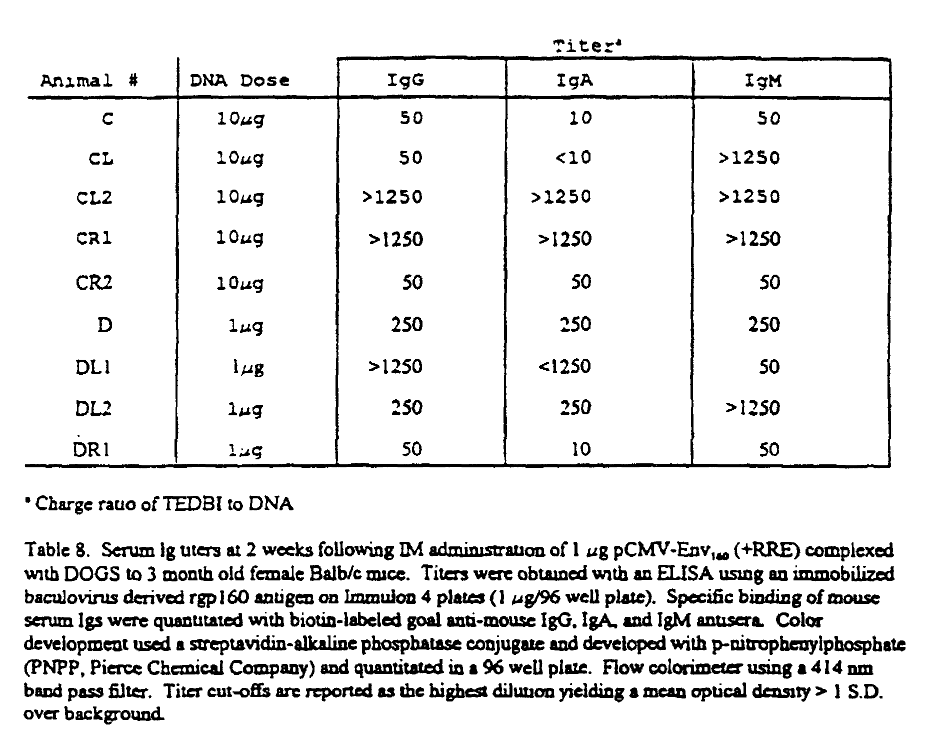

### General Experimental Design

Based on the comparative data on induction of mucosal responses by three different methods of genetic immunization (mucosal cell transfection, holistic vaccination, and HIPGV), HIPGV appears to be the most effective. HIPGV uses facilitated transfection of muscle cells in which the HIV envelope sequences are expressed on the cell surface in a manner analogous to HIV-infected cells. The hormonal adjuvant - 1,25(OH)₂D3 - appears to act as a molecule switch that results in the trafficking of HIV-specific B cells committed as IgA secreting plasma cells to the mucosa in a common mucosal response. It is reasonable to hypothesize that a similar phenomenon exists for T cells destined to mediate cytotoxic functions. Hoffman-LaRoche has provided 5 mg of synthetic 1,25(OH)₂D3 which is identical to that used by Dayne and Arranes. This is sufficient for 5000 genetic immunizations in mice with the current protocol. Nine HIV-envelope genetic vaccines have been constructed (Table 3) under control of the CMV early promoter. CMV_env160 contains the full length gp160 including leader sequence, gp120/gp41 proteolysis site, intact RRE, enhancing domain, and the membrane anchor sequence. CMV_env1R160 is identical to CMV_env160 except that it contains a deletion 4 amino acid deletion at the proteolysis that renders the gp160 molecule resistant to proteolysis. The remainder (n=7) of the HIV-envelope genetic constructs are identical to CMV_env160 except that they contain deletions or point mutations in the enhancing domain. Depending on their effectiveness as genetic immunogens and the effectiveness of CMV_env1R160, one or more of these enhancing domain mutations will be produced into the proteolysis resistant mutant. By inhibiting proteolysis and gp120 shedding from transfected cells, the responses to gp120 can be bolstered while retaining the magnitude of gp41 responses. By introducing mutations of the enhancing domain into such a construct, beneficial functional responses to both gp120 and gp41 can be maximized while minimizing deleterious functional responses to the enhancing domain. Although the initial efforts which focus on the NL4-3 isolate envelope sequences will allow exploration of the mechanisms and kinetics of genetically induced mucosal immune responses, gp160 envelope sequences derived from primary isolates of newly mucosally infected subjects can be used. These considerations have led to the following specific aims and their experimental design:

### 1) Quantitation of IgA in parotid secretions

It is desirable quantitate the concentration of IgA in parotid, jejunal, colonic, rectal and uterine/vaginal secretions versus systemic Ig as a function of time and concentration of pCMV vectors containing various HIV-env_{NL4-} ₃ inserts. Using 1µg 1,25(OH)₂D3 as the established hormonal adjuvant, parotid IgA and IgG titers are monitored to establish the peak and duration of primary IM genetic vaccination. Based on this data, a larger animal cohort is established to allow animal sacrifice over time with quantitation of jejunal, colonic, and uterine/vaginal mucosal IgA concentrations as well as parotid. The recently published technique from the Neutra lab (188) in which a Polyfiltronic wick is used to adsorb mucosal secretions (>90% IgA recovery) will be used. Adsorbed Ig is eluted with PBS and centrifuged at 16,000 X g to ensure maximal extraction. Eluted IgA representing primarily sIgA and IgG will be quantitated with an ELISA assay (see Specific Methods).

### 2) Enhancement of mucosal sIga responses

Methods to enhance rectal and uterine/vaginal mucosal sIgA responses following primary HIPGV against HIV-envelope can be routinely evaluated. Once the optimal conditions for obtaining mucosal immune responses with primary HIPGV are defined, boosting rectal and uterine/vaginal mucosal responses by local (direct mucosal) administration of antigen (gp160) and by facilitated transfection of rectal and uterine/vaginal mucosa with the original genetic immunogen in a manner analogous to the original airway facilitated transfection method can be done. The rationale in this experimental approach is that either protein antigen or antigen produced locally by local secondary genetic vaccination will result in a local amnestic response.

### 3) Quantitation of functional Ab activity

Functional antibody activities in mucosal secretions following HIPGV and local amnestic induction can be quantified. Mouse serum contains a powerful HIV neutralizing activity that can be inactivated by heating at 56°C for 1/2 hour. After establishing whether mucosal neutralizing activities are present in naive animals, the standard neutralization protocol (185) to mouse mucosal secretions is adapted accordingly. p24 and RT assays in primary human PBMC cultures can be used to establish neutralization titers. The standard enhancing assay using human complement can be adapted to mucosal secretions. Binding activity of mucosal IgA and IgG to a 35 amino acid peptide which has been used previously that spans the enhancing domain (145) can be used to demonstrate abscence of enhancing epitopes.

### 4) Evaluation of lymphocyte subpopulations

As a function of time following primary HIPGV, lymphocyte subpopulations in draining lymph nodes and at mucosal sites (i.e., intraepithelial and lamnia propria), lymphocytes with surface HIV-envelope receptors, regulatory proteins, and relevant surface adhesion molecules are evaluated. This data is relevant to understand the trafficking of HIV-env specific lymphocytes induced by 1,25(OH)₂D3 and the B versus T cell responses responding to HIPGV. The relative densities of B cells, CD4, and CD8 cells as a function of HIPGV in formalin fixed tissues can be determined. Similarly, mucosal and nodal lymphocytes can be examined for the presence of adhesion molecules known to be involved in mucosal homing such as VLA-4α (189, 190). The method of Ni (192) will be used with fresh tissues to isolate mucosal lymphocytes and subject them to standard analysis by flow cytometry.

### 5) Cellular cytotoxicity against HIVenv

Cellular cytotoxic activities against HIV-envelope expressing Balb/c fibroblasts from spleen and mucosal sites are quantitated. The point in this specific aim is to monitor CTL for effects secondary to the mutagenic constructs. Although CTL epitopes in mice and humans will generally not be identical, this assay insures that the various immunogens will not inhibit CTL development when applied to humans. As detailed in summary of results, an assay is taught which quantitates splenocyte CTL activity. Using the method of Ni et al. sufficient lymphocytes can be isolated from mucosal sites to be able to apply this assay to ascertain whether CTLs are enhanced by vitamin D3 present in mucosal sites.

### 6) Toxicity of DNA immunogens

The potential toxicity of the DNA immunogens with respect to the persistence of the genetic construct in muscle as a function of time and whether the genetic constructs can be detected in non-muscle tissues (gonads, liver, spleen, lung, etc.) can be determined. A basic impediment to FDA approval of any Phase I genetic immunogen is animal data establishing evidence of safety. Of particular importance to the FDA is proof that the genetic immunogen is localized to the vaccination site and cannot be found in non-muscle sites, especially gonadal sites. A Master Vector File will be established with the FDA for the purpose of obtaining RAC and FDA approvals.

Mice, rats, guinea pigs, and rabbits will be monitored for the development of antibodies to plasmid vector DNA using an ELISA format in which DNA is adhered to Immulon plates as previously described for peptide antigens and albumin blocked wells exposed to serum from transfected animals. Antibodies binding to DNA will be detected by anti-mouse (or rat, guinea pig, or rabbit) Ig conjugated to alkaline phosphatase. Quantitation will be based on enzyme yields minus control animal enzyme yields under conditions of substrate excess (*i.e*., to yield zero order kinetics). Mice will be the primary animal for safety evaluation. Depending on FDA advice, another species will be chosen as a second toxicity testing target. Weights during HIPGV and standard gross and histopathology at the conclusion of the experimental protocol will be conducted on all the mice. In addition PCR amplification for the HIV-envelope immunogens will be performed on lung, spleen, liver, and gonadal tissues. This procedure is well established with reproducible amplification of a 1200 bp sequence. Identification is made by standard agarose electrophoresis and ethidium bromide staining of the amplified DNA plus base-ladder size markers. A FDA approved anti-fos gene therapy Phase I study in metastatic breast carcinoma of Holt can be used as a model.

### 7) Additional immunogens

To construct more relevant HIV-envelope genetic immunogens from primary isolates based on the data generated with pNL4-3 HIV-envelope genetic immunogens, HIV envelope sequences are routinely PCR cloned into p-Alter or p-CMV vector by 5'-extensions of restriction sites for cloning into MCS of the plasmid vectors. Cloning envelope sequences from cloned primary isolates which can be obtained as they become available.

### Systemic Antibody Analysis

a. Ig Titers. Serum titers of antibodies against the env proteins of HIV-1 are quantitated with an ELISA procedure using baculovirus derived rgp160 as the plate immobilized ligand. 10 ng rgp160 (AMAC, Inc.) in 50 µg coating buffer (0.1 M Na carbonate, pH 9.5). is immobilized on each well of a 96 well Immulon 4 microtiter plate for hour at 37°C. The plate is washed with PBS-0.15% Tween20 x3 and is then blocked with PBS-1% BSA-0.15% Tween20 at 300 µg per well for 1 hour at RT and then washed x3 with PBS-0.15% Tween20. Serum or mucosal secretions are serially diluted in PBS in triplicate in a separate plate and 50 µg of each well transferred to corresponding wells of a gp160 ligand plate and the following sequence is followed. Incubate at 37°C for 1 hour using a parafilm cover. Wash with PBS-1% FCS-0.05% NaN₃ x5. Incubate each well with a predetermined dilution of biotin conjugated anti-mouse IgG, IgA, or IgM. Incubate at 37°C for 1 hour with cover. Wash with PBS-1% FCS-0.05% NaN₃x5. Follow with 50 µg streptavidin-alkaline phosphatase conjugate (1:200 in PBS-1% BSA-0.15% Tween20) for 1 hr at 37°C with cover. Wash x5 with PBS-1% FCS-0.05% NaN₃. Color is developed with p-nitrophenyl phosphate in glycine buffer at pH 9.6. The color yield is measured on a Flow microtiter colorimeter using a 405 nm filter. Background is routinely <0.23 with a reproducibility <±0.005). End point titer is the highest dilution of serum or secretion yielding a color yield >150 over background (n=3).

### Mucosal Antibody Analysis

a. Analysis of jejunal, colonic, and uterine/vaginal secretions for IgA/IgG: Secretions will be collected on Polyfiltronics absorbent wick filters as described recently by the Neutra lab (188). IgA and IgGs eluted from the wicks will be assayed by the present ELISA procedure.
b. Immunocytochemistry: rgp160 conjugated with biotin (AMAC, Inc) was used as a specific probe for tissue bound antibodies/receptors generated by the present genetic immunization procedures. The procedure can be used for either frozen or formalin fixed tissues. Thin sections are incubated with 250 µl of the biotinylated ligand (10 ng/ml) for 1/2 hour at RT, washed x2 with PBS. 250 µl of streptavidin-β galactosidase (Kirkegaard & Perry Labs) in 100 mM Tris, pH 7.4 is applied to the section for 1/2 hour at RT, washed x2 with PBS. Color is developed at pH 7.6 using X-Gal (5-bromo-4-chloro-3 indoyl-β)-galactopyranoside as substrate (Histomark kit from K&P Labs) which yields an azure blue precipitate at the site of bound enzyme. Typically an adjacent, non-ligand exposed section is used as a control for non-specific product deposition as well as sections from a naive animal exposed to ligand.

### Literature Cited

1. Ulmer JB, Donnelly JJ, Parker SE, Rhodes GH, Felgner PL, Dwarki VJ, Gromkowski SH, Deck RR, DeWitt CM, Friedman A, Hawe LA, Leander KR, Martinez D, Perry HC, Shiver JW, Montgomery DL, and Liu MA. Heterologous protection against influenza by injection of DNA encoding a viral protein. Science 1993, 259:1745-1749.
2. Dayne RA, and Aranes BA. The development of effective vaccine adjuvants employing natural regulators of T-cell lymphokine production *in vivo*. Ann NY Acad Sci 1994, 730:144-161.
3. Wolff JA, Malone RW, Williams P, Chong W, Acsadi G, Jani A, and Feigner PL. Direct gene transfer into mouse muscle *in vivo.* Science 1990, 247:1465-1469.
4. Lin H, Parmacek MS, Morle G, Bolling S, and Leiden JM. Expression of recombinant genes in myocardium in vivo after direct injection of DNA. Circulation 1990, 82:2217-2221.
5. Acsadi G, Jio S, Jani A, Duke D, Williams P, Chong W, and Wolff JA. Direct gene transfer and expression into rat heart *in vivo*. New Biologist 1991, 3:71-81.
6. Kitsis RN, Buttrick PM, McNally EM, Kaplan ML, and Leinwald LA. Hormonal modulation of a gene injected into rate heart *in vivo*. Proc Natl Acad Sci USA 1991, 88:4138-4142.
7. Hansen E, Fernandes, K, Goldspink G, Butterworth P, Umeda PK, and Chang K-C. Strong expression of foreign genes following direct injection into fish muscles. FEBS Lett 1991, 290:73-76.
8. VonHarsdorf R, Schott RJ, Shen YT, Vatner SF, Mahdavi V, and Nadlginard B. Gene injection into canine myocardium as a useful model for studying gene-expression in the heart of large animals. Circ Res 1993, 72:688-695.
9. Gal D. Weir L. LeClerc G, Pickering JG, Hogan J, and Isner JM. Direct myocardial transfection in 2 animals models: evaluation of parameters affecting gene-expression and percutaneous gene delivery. Lab Invest 1993, 68:18-25.
10. Cox, GJM, Zamb TJ, and Babiuk LA. Bovine herpesvirus 1: immune responses in mice an cattle injected with plasmid DNA. J Virol 1993, 67:5664-5667.
11. Jiao S, Williams P, Berg RK, Hodgeman BA, Liu L, Repetto G, and Wolff Ja. Direct gene transfer into nonhuman primate myofibers *in vivo.* Hum Gene Ther 1992, 3:21-33.
12. Davis HL, Demeneix BA, Quantin B, Coulombe J, and Whalen RG. Plasmid DNA is superior to viral vectors for direct gene transfer into adult mouse skeletal muscle. Hum Gene Ther 1993, 4:733-740.
13. Yankauckas MA, Morrow JE, Parker SE, Abai A, Rhodes GH, Dwarki VJ, and Gromkowski SH. Long-term anti-nucleoprotein cellular and humoral immunity is induced by intramuscular injection of plasmid DNA containing NP gene. DNA Cell Biol 1993, 12:771-776.
14. Fynan EF, Robinson HL, and Webster Rg, Use of DNA encoding influenza hemagglutinin as an avian influenza vaccine. DNA Cell Biol 1993, 12:785-789.
15. Montgomery DL, Shiver JW, Leander KR, Perry HC, Friedman A, Martinez D, Ulmer JB, Donnelly JJ, and Liu MA. Heterologous and homologous protection against influenza A by DNA vaccination: optimization of DNA vectors. DNA Cell Biol 1993, 12:777-783.
16. Wang B, Ugen KE, Srikantan V, Agadjanyan MG, Dang K, Refaeli Y, Sato AI, Boyer J, Williams WV, and Weiner DB. Gene inoculation generates immune responses against human immunodeficiency virus type 1. Proc Natl Acad Sci USA 1993, 90:4156-4160.
17. Wang B, Boyer J, Srikantan V, Coney L, Carrano R, Phan C, Merva M, Dang K. Agadjanan M, Gilberg L. Ugen KE, Williams WV, and Weiner DB. DNA inoculation induces neutralizing immune responses against human immunodefieiency virus type 1 in mice and nonhuman primates. DNA Cell Biol 1993, 12:799-805.
18. David HL, Michel M-L, and Whalen RG. DNA-based immunization induces continuous secretion of hepatitis B surface antigen and high levels of circulating antibody. Hum Mol Genet 1993, 2:1847-1851.
19. David HL, Whalen RG, and Demeneix BA. Direct gene transfer into skeletal muscle in vivo: factors affecting efficienty of transfer and stability of expression. Hum Gene Ther 1993, 4:151-159.
20. Johnson RP, Hammond SA, Trocha A, Siliciano RF, and Walker BD. Epitope specificity of MHC restricted cytotoxic T lymphocytes induced by candidate HIV-1 vaccine. AIDS Res Hum Retroviruses 1994, 10:573-575.
21. Hui KM, Sabapathy TK, Oei AA, and Chia TF. Generation of allo-reactive cytotoxic T lymphocytes by particle bombardment-mediated gene transfer. J Immunol Methods 1993, 171:147-155.
22. Haynes JR, Fuller DH, Eisenbraun MD, Ford MJ, and Pertner TM. Accell® particle-mediated DNA immunization elecits humoral, cytotoxic, and protective immune responses. AIDS Res Human Retroviruses 1994, 10:543-545.
23. Eisenbraun MD, Fuller DH, and Haynes Jr. Examination of parameters affecting the elicitation of humoral immune responses y particle bombardment-mediated genetic immunization. DNA Cell Biol 1993, 12:791-797.
24. Tang D-C, DeVit M, and Johnston SA. Genetic immunization is a simple method for eliciting an immune response. Nature 1992, 356:152-154.
25. O'Hagan DT. Oral immunization and the common mucosal immune system. In *Novel Delivery Systems for Oral vaccines,* Derek T. O'Hagan, ed. CRC Press, Cleveland, Ohio, 1994, pp. 1-24.
26. Service RF. Triggering the first line of defense. Science 1994, 265:1522-1524.
27. Suharyona, Simanjuntak C, Witham N, Punjabi N, Heppner DG, Losonsky G, Totosudirjo H, Rifai AR, Clemens J, Lim YL, Burr D, Wasserman SS, Kaper J, Sorenson K, Cryz S, and Levine MM. Safety and immunogenicity of single-dose oral cholera vaccine CVD 103 HgR in 5-9 year old children. Lancet 1992, 340:689:694.
28. Desrosiers RC. Hiv with multiple gene deletions as a live attenuated vaccine for AIDS. AIDS Res Hum Retrovir 1992, 8:411-421.
29. Cohen J. AIDS Vaccines. At conference, hope for success is further attenuated. Science 1994, 266:1154.
30. Padian NS. Heterosexual transmission of acquired immune deficiency syndrome: international perspective and national projections. Rev Infect Dis 1987; 9:947-960.
31. Piot P, Plummer F, Mhalu FS, Lamboray JL, Chin J, and Mann JM. AIDS: an international perspective. Science 1988, 239:573-579.
32. Johnson AM. Heterosexual transmission of human immunodeficiency virus. Brit Med J 1988, 296:1017-1029.
33. Hospedales J. Heterosexual spread of HIV infection. Rev Infect Dis 1989, 11:663-665.
34. deSchryver A, and Meheus A. Epidemiology of sexually transmitted diseases: the global picture. Bull WHO 1990, 68:639-654.
35. Alexander NJ. Sexual transmission of human immunodeficiency virus: virus entry into the male and female genital trace. Fertil Steril 1990, 54:1-18/
36. Miller C, and Gardner MB. AIDS and mucosal immunity: usefulness of the SIV macaque model of genital mucosal transmission. J AIDS 1991, 4:1169-1172.
37. McGhee JR, and Mestecky J. The mucosal immune system in HIV infection and prospects for mucosae immunity to AIDS. AIDS Res Rev 1992, 2:289-312.
38. Forrest BD. The need for consideration of mucosal immunity in vaccine approaches to AIDS. Vaccine Res 1992, 1:137.
39. Padian ND, Shiboski SC, and Jewell NP. Female to male transmission of human immunodeficiency virus. J Amer Med Assoc 1991, 266:1664-1667.
40. Forrest BD. Women, HIV, and mucosal immunity. Lancel 1991, 337:835-836.
41. Miller CJ, McGhee Jr, and Gardner MB. Mucosal immunity, HIV transmission and AIDS. Lab Invest 1992, 68:129-145.
42. Mestesky J, Kutteh WH, and Jackson S. Mucosal immunity in the female genital tract: relevance to vaccination efforts against the Human Immunodeficiency Virus. AIDS Res Human Retroviruses 1994, 10:511-520.
43. Miller CJ, Alexander NJ, Sutjipto S, Lackner AA, Hendrickx AG, Lowenstine LJ, Jennings M, and Marx PA. Genital musocal transmission of simian immunodeficiency virus: animal model for heterosexual transmission of human immunodeficiency virus. J Virol 1989, 63:4277-4284.
44. Lehner T, Bergmeier LA, Panagiotidi C, Tao L, Brookes R, Klavinskis LS, Walker P, Walker J, Ward RG, and Hussain L: Induction of mucosal and systemic immunity to a recombinant simian immunodeficiency viral protein. Science 1992, 258:1365-1369.
45. Lehner T, Brookes R, Panagiotid C, Tao L, Klavinskis LS, Walker J, Walker P, Ward R, Hussain L, Gearing AJH, Adams SE, and Bergmeier LA. T- and B-cell functions and epitope expression in nonhuman primates immunized with simian immunodeficiency virus antigen by the rectal route. Proc Natl Acad Sci USA 1993, 90:8638-8642.
46. Marx PA, Compans RW, Gettie A, Staas JK, Gilley RM, Mulligan MJ, Yamschikov GV, Chen D, and Eldridge JH. Protection against vaginal SIV transmission with microencapsulated vaccine. Science 1993, 260:1323-1327.
47. Mestecky J, and McGhee JR. Immunoglobulin A (IgA): molecular and cellular interactions involved in IgA biosynthesis and immune response. Adv Immunol 1987, 40:153-245.
48. Melnick JL. Enteroviruses: Polioviruses, Coxsackiviruses, Echoviruses, and newer Enterviruses. In, *Virology.* Bernard N. Fields and David M. Knipe, eds in chief. Raven Press, New York, NY 1990, 549-605.
49. Mestecky J, Lue C, and Russell MW. Selective transport of IgA: cellular and molecular aspects. Gastroenterol Clin North Amer 1991, 20:441-471.
50. Mestecky J. The common mucosal immune system and current strategies for induction of immune responses in external secretions. J Clin Immunol 1987, 7:265-276.
51. Papsidero, LD, Sheu, M, and Ruscetti, FW. Human immunodeficiency virus type 1-neutralizing monoclonal antibodies which reach with p17 core protein: characterization and epitope mapping. J Virol 1989, 63:267-272.
52. Myers G, Rabson AB, Berzofsky JA, Smith RF and Wont-Stall F. Human Retroviruses and AIDS. Los Alamos National Laboratory, Los Alamos NM 1990.
53. Ho, DD, Kaplan JR, Rackauskas IE and Gurney ME. Second conserved domain of gp120 is important for HIV infectivity and antibody neutralization. Science 1988, 239:1021-1023.
54. Ho DD, Sarngadharan MG, Hirsch MS, Schooley RT, Rota Tr, Kennedy RC, Chanh TC, Sato VL. Human immunodeficiency virus neutralizing antibodies recognize several conserved domains on the envelope glycoproteins. J. Virol 1987, 61:2024-2028.
55. Rusche JR, Javaherian K, McDanal C, Petro J, Lynn DL, Grimaila R, Langlois A, Gallo RC, Arthur LO, Fischinger PJ, Bolognesi DP, Putney SD, and Matthews, TJ. Antibodies that inhibit fusion of human immunodeficiency virus-infected cells bind a 24-amino acid sequence of the viral envelope, gp120. Proc Natl Acad Sci Usa 1988, 85:3198-3202.
56. Palker TJ, Matthews TJ, Langlois A, Tanner ME, Martin ME, Scearce RM, Kim JE, Berzofsky JA, Golognesi DP, Haynes BF. Polyvalent human immunodeficiency virus synthetic immunogen comprised of envelope gp120 T helper cell sites and B cell neutralization epitopes. J Immunol 1989, 142:3612-3619.
57. Palker TJ, Clark ME, Langlois AJ, Matthews TJ, Weinhold KJ, Randall RR, Bolognesi DP, Haynes BF. Type-specific neutralization of the human immunodeficiency virus with antibodies to env-encoded synthetic peptides. Proc Natl Acad Sci USA 1988, 85:1932-1936.
58. Kenealy WR, Matthews TJ, Ganfield M-C, Langlois AJ, Waselefsky DM, and Petteway Sr., Jr. Antibodies from human immunodeficiency virus-infected individuals bind to a short amino acid sequence that elicits neutralizing antibodies in animals. AIDS Res Human Retrovirus 1989, 5:173-181.
59. Javaherian K, Langlois AJ, McDanal C, Ross KL, Eckler LI, Jellis CL, Profy AT, Rusche JR, Bolognesi DP, Putney SD. Principal neutralizing domain of the human immunodeficiency virus type 1 envelope protein. Proc Natl Acad Sci USA 1989, 86:6768-6772.
60. Chanh TC, Dreesman GR, Kanda P, Linette GP, Sparrow JT, Ho DD, Kennedy RC. Induction of anti-HIV neutralizing antibodies by synthetic peptides. EMBO J 1986, 5:3065-3071.
61. Schrier, RD, Gnann, JW, Jr., Langlois AJ, Shriver K, Nelson JA, and Oldstone MB. B- and T-lymphocyte responses to an immunodominant epitope of human immunodeficiency virus. J Virol 1988, 62:2561-2536.
62. Evand DJ, McKeating J, Meredith JM, Burke KL, Katrak K, John A, Ferguson M, Minor PD, Weiss RA, Almond JW. An engineered poliovirus chimaera elicits broadly reactive HIV-1 neutralizing antibodies. Nature 1989, 339:385,388.
63. Sun NC, HO DD, Sun CR, Liou RS, Gordon W, Fung MS, Li XL, Ting Rc, Lee Th, Chang NT. Generation and characterization of monoclonal antibodie sto the putative CD4-binding domain of human immunodeficiency virus type 1 gp120. J Virol 1989, 63:3579-3585.
64. Goudsmit J, Debouck C. Meloen RH, Smit L, Bakker M, Asher DM, Wolff AV, Gibbs CJ, jr., Gajdusek DC. Human immunodeficiency virus type 1 neutralization epitope with conserved architecture elicits early type specific antibodies in experimentally infected chimpanzees. Proc Natl Acad Sci USA 1988, 85:4478-4482.
65. Meloen, RH, Liskamp RM, and Goudsmit, J. Specificity and function of the individual amino acids of an important determinant of human immunodeficiency virus type 1 that induces neutralizing activity. J Gen Virol 1989, 70:1505-1512.
66. Marthas ML, Sutjipto S, Higgins J, Lohman B, Torten J, Luciw PA, Marx PA, Pedersen NC. Immunization with a live, attenuated simian immunodeficiency virus (SIV) prevents early disease but not infection in rhesus macaques challenged with pathogenic SIV. J Virol 1990, 64:3694-3700.
67. Goudsmit J, Krone WJ, and Wolfs, TFW. Genomic divergence within the coding sequence for the principal neutralization epitope of HIV-1. Quatrième Collogue des Cent Gardes, L'Institut Pasteur, Marnes-La-Coquette, France, 1989, 55-60.
68. Putney S, Larosa G, and Matthews T. The principal neutralizing determinant of HIV-1. Quatrieme Colloque des Cent Gardes, L'Institut Pasteur, Marnes-la-Coquette, France, 1989, 189-193.
69. Bolognesi, DP. General Features of the V3 neutralization loop of HIV. Quatrième Colloque des Cent Gardes, L'Institut Pasteur, Marines-La-Coquette, France, 1989, 181-188.
70. Carrow E, Vujcic L, Hendry R, Galvao F, Halsey N, Boulos R, and Quinnau G. Geographically diverse antibody responses to the variable region 3 (V3) neutralizing epitope of the HIV-1 envelope. VI Intl Conf on AIDS, San Francisco, CA 1990, #S.A. 281.
71. Putney S, Larosa G, Lewis J, Profy A, Weinhold K, Matthews T, Boswell N, Dreesman G, Holley H, Karpus M, Emini E, and Bolognesia D. The HIV-1 principal neutralizing determinant contains conserved sequences and structural elements. VI Intl Conf on AIDS, San Francisco, CA 1990, #F.A. 211.
72. Katzenstein DA, Vujcic LK, Latif A, Boulos R, Halsey NA Quinn TC, Rastogi SC, Quinnan GV, Jr. Human immunodeficiency virus neutralizing antibodies in sera from North Americans and Africans. J Acq Imm Def Syn 1990, 3:810-816.
73. McKeating JA, Gow J, Goudsmit J, Pearl LH, Mulder C, and Weiss RA. Characterization of HIV-1 neutralization escape mutants. AIDS 1989, 3:777-784.
74. Kuiken CL, DeJong J-J, Tersmette M, Smit L, Nara PL, and Goudsmit J. Changes in a neutralization domain of sequential isolate in experimental and natural HIV infection. VI Intl Conf on AIDS, San Francisco, CA 1990, #S.A. 278.
75. Wolfs TFW, Goudsmit J, Dejong J-J, Kuiken CL, Van den Berg, H and Krone WJA. Individual selection for genetic variants of the V3 major neutralizing domain. VI Intl Conf on AIDS, San Francisco, CA 1990, #S.A.279.
76. Vujcic L, Carrow E, Seamon K, and Quinnan, G. Periodic changes in neutralization epitopes of HIV-1 involve variable region 3. VI Intl Conf on AIDS, San Francisco, CA 1990, #S.A.277.
77. Robert-Guroff M, Reitz, MS, Jr, Robey, WG, and Gallo RC. In vitro generation of an HTLV-III variant by neutralizing antibody. J Immunol 1986, 137:3306-3309.
78. Reitz MS, Jr., Wilson C, Naugle C, Gallo RC, and Robert-Guroff, M. Generation of a neutralization resistant variant of HIV-1 is due to selection for a point mutation in the envelope gene. Cell 1988, 54:57-63.
79. Albert J., Abrahamsson B, Nagy K. Aurelius E, Gaines H, Nystrom G, Fenyo EM. Rapid development of isolate-specific neutralizing antibodies after primary HIV-1 infection and consequent emergence of virus variants which resist neutralization by autologous sera. AIDS 1990, 4:107-112.
80. Wilson C, Reitz MR Jr., Aldrich K, Klasse PJ, Blomberg J, Gallo RC, Robert-Guroff M. The site of an immune-selected point mutation in the transmembrane protein of human immunodeficiency virus typel does not constitute the neutralization epitope. J Virol 1990, 64:3240-3248.
81. Nara PL, Smit L, Dunlop N, Hatc W, Merges M, Waters D, Kelliher J, Gallo RC, Fischinger PJ, Goudsmit J. Emergence of viruses resistant to neutralization by V3-Emergence of viruses resistant to neutralization by V3-specific antibodies in experimental human immunodeficiency virus type 1 IIIB infection of chimpanzees. J Virol 1990, 64:3779-3791.
82. Weiss, RA, Clapham PR, Cheingsong-Popov, R, Daigleish AG, Carne CA, Weller ND and Tedder, RS. Neutralization of human T-lymphotropic virus type III by sera of AIDS and AIDS-risk patients. Nature 1985, 316:69-72.
83. Weiss, RA, Clapham PR, Weber JN, Dalgleish, AG, Lasky LA, and Berman PW. Variable and conserved neutralization antigens of human immunodeficiency virus. Nature 1986, 324:572-575.
84. Berkower I, Smith GE, Giri C, and Murphy D. Human immunodeficiency virus 1: predominance of a group-specific neutralizing epitope that persists despite genetic variation. J Exp Med 1989, 170:1681-1695.
85. Nara, PL, Garrity RR, and Goudsmit J, Neutralization of HIV-1: a paradox of humoral proportions. FASEB J. 1991, 5:2437-2455.
86. Ho, DD, Li ZL, Daar ES, Mondgil T, Sun NC, Holton D, and Robinson JE. A neutralizing human monoclonal antibody (HMAb) identifies an epitope within the putative CD4-binding domain (CD4-BD) of HIV-1 gp120. VI Intl Conf on AIDS, San Francisco, CA 1990, #Th.A.76.
87. Weiss RA, Clapham PR, McClure MO, McKeating JA, McKnight A, Daigleish AG, Sattentau QJ, Weber JN. Human immunodeficiency viruses: neutralization and receptors. J Acq Imm Def Dyn 1988, 1:536-541.
88. Larkin M, Childs RA, Matthews TJ, Thiel S, Mizuochi T, Lawson AM, Savill JS, Haslett C, Diaz R, Feizi T. Oligosaccharide-mediated interactions of the envelope glycoprotein gp120 of HIV-1 that are independent of CD4 recognition. AIDS 1989, 3:793-798.
89. Qureshi, NM, Coy DH, Garry RF, and Henderson, LA> Characterization of a putative cellular receptor for HIV-1 transmembrane glycoprotein using synthetic peptides. AIDS 1990, 4:553-558.
90. Broliden, PA, Ljunggren K, Hinkula J, Norrby E., Akerblom L, and Wahren B. A monoclonal antibody to human immunodeficiency virus type 1 which mediates cellular cytotoxicity and neutralization. J Virol 1990, 64:936-940.
91. Dowbenko D, Nakamura G, Fennie C, Shimasaki C, Riddle L, Harris R, Gregory T, Lasky L. Epitope mapping of the human immunodeficienty cirus type 1 gp 120 with monoclonal antibodies. J Virol 1988, 62:4703-4711.
92. Desgranges C, Boyer V, Souche S, Sprecher S, Burney A, Gallo RC, Bernard J, Reveil B, Zagury D. Monoclonal antibodie sto HIV in a non-infected, immunised volunteer. Lancet 1988, i:935-936.
93. Thali M, Olshevsky U, Furman C, Gabuzda D, Posmer M, and Soidroski J. Characterization of a discontinuous human immunodeficiency virus type 1 gp 120 epitope recognized by a broadly reactive neutralizing human nomoclonal antibody. J. Virol. 1991, 65:6188-6193.
94. Hansen JE, Clausen H, Nielsen C, Teglbjaerg LS, Hansen LL, Nielsen CM, Dabelsteen E, Mathiesen L, Hakomori SI, Nielsen JO. Inhibition of human immunodeficiency virus (HIV) infection in vitro by anticarbohydrate monoclonal antibodies: peripheral glycosylatin of HIV envelope glycoprotein gp120 may be a target for virus neutralization. J Virol 1990, 64:2833-2840.
95. Miller, WEG, Schröder HC, Reuter P, Maidhof A, Uhlenbruck G, and Winkler I. Polyclonal antibodies to mannan from yeast also recognize the carbohydrate structure of gp120 of the AIDS virus: an approach to raise neutralizing antibodies to HIV-1 infection in vitro. AIDS 1990, 4:159-162.
96. Gruters RA, Neefjes JJ, Tersmette M, De Goede RE, Tulp A, Huisman HG, Miedema F, Ploegh H1. Interference with HIV-induced syncytium formation and viral infectivity by inhibitors of trimming glucosiadase. Nature 1987, 330:74-77.
97. Walker BD, Kowalski M, Goh WC, Kozarsky K, Krieger M, Rosen C, Rohrschneider L, Haseltine WA, Sodroski J. Inhibition of human immunodeficiency virus syncytium formation and virus replication by castanospermine. Proc Natl Acad Sci USA 1987, 84:8120-98124.
98. Sunkara, PS, Bowlin Tl, Liu PS and Sjoerdsma A. Antiretroviral activity of castanospermine and deoxynojirimycin, specific inhibitors of glycoprotein processing. Biochem Biophys Res Commun 1987, 148:206-210.
99. Tyms AS, Berrie Em, Ryder Ta, Nash RJ, Hegarty MP, Taylor Dl, Mobberley MA, Davis JM, Bell EA, Jeffries DJ. Castanospermine and othe rplant alkaloid inhibitors of glucosidase activity block the growth of HIV. Lancet 1987, ii:1025-1026.
100. Montefiori DC, Robinson WE Jr., and Mitchell WM. Role of protein N-glycosylation in pathogenesis of human immunodeficiency virus type 1. Proc Natl Acad Sci USA 1988, 85:9248-9252.
101. Fenouillet, E, Gluckman, JF, and Bahraoui E. Role of Nlinked glycans of envelope glycoproteins in infectivity of human immunodeficiency virus type 1. J Virol 1990, 64:2841-2848.
102. Matthews TJ, Weinhold KJ, Lyerly HK, Langlois, AJ, Wigzell H, and Bolognesi D. Interaction between the human T-cell lymphotropic virus type IIIB envelope glycoprotein gp120 and the surface antigen CD4: role of carbohydrate in binding and cell fusion. Proc Natl Acad Sci USA 1987, 84:5424-5428.
103. Lifson J, Coutre S, Huang E, and Engleman E. Role of envelope glycoprotein carbohydrate in human immunodeficiency virus (HIV) infectivity and virus-induced cell fusion. J Exp Med 1986, 164:2101-2106.
104. Robinson, WE Jr., Montefiori, DC and Mitchell WM. Evidence that mannosyl residues are involved in human immunodeficiency virus type 1 (HIV-1) pathogenesis. AIDS Res Human Retrovirus 1987, 3:265-282.
105. Ezekowitz, RAB, Kuhlman M, Groopman Je, and Bryn RA. A human serum mannose-binding protein inhibits in vitro infection by the human immunodeficiency virus. J Exp Med 1989, 169:185-196.
106. Müller, WEG, Renneisen, K. Kreuter, MH, Schroder, HC, and Winkler I. The D-mannose-specific lectin from Gerardia savaglia blocks binding of human immunodeficiency virus type 1 to H9 cells and human lymphocytes in vitro. J Acq Imm Def Syn 1988, 1:453-348.
107. Haigwood NL, Shuster JR, Moore GK, Lee H, Skiles PV, Higgins KW, Barr Pj, George-Nascimento C, Steimer KS. Importance of hypervariable regions of HIV-1 gp120 in the generation of virus neutralizing antidoies. AIDS Res Human Retrovirus 1990, 6:855-869.
108. Matsushita S, Robert-Guroff M, Rusche J, Koito A, Hattori T, Hoshino H, Javaherian K, Takatsuki K, Putney S. Characterization of a human immunodeficiency virus neutralizing monoclonal antibody and mappy of the neutralizing epitope. J Virol 1988, 62:2107-2114.
109. Skinner MA, Ting R, Langlois AJ, Weinhold KJ, Lyerly HK, Javaherian K. Matthews TH. Characteristics of a neutralizing monoclonal antibody to the HIV envelope glycoprotein. AIDS Res Human Retrovirus 1988, 4:187-197.
110. Robinson WE Jr, Montefiori DC and Mitchell WM. A human immunodeficiency virus type 1 (HIV-1) infection-enhancing factor in seropositive sera. Biochem Biophys Res Commun 1987, 149:693-699.
111. Robinson WE Jr, Montefiori DC, and Mitchell Wm. Antibody-dependent enhancement of Human Immunodeficiency Virus Type 1 infection. Lancet 1988 i:790-794.
112. Porterfield JS. Antibody-dependent enhancement of viral infectivity. Adv Virus Res 1986, 31:335-355.
113. Halstead SB. Pathogenesis of dengue: challenges to molecular biology. Science 1988, 238:476-481.
114. Halstead SB, and O'Rourke EJ. Antibody-enhanced dengue virus infection in primate leukocytes. Nature 1977, 265:739-741.
115. Kliks SC, Nisalak A, Brandt WE, Wahl L, and Burke DS. Antibody-dependent enhancement of dengue virus growth in human monocytes as a risk factor for dengue hemorrhagic fever. Am J Trop Med Hyg 1989, 40:444-451.
116. Hawkes RA. Enhancement of the infectivity of arboviruses by specific antisera produced in domestic fowls. Aust J Exp Biol Med Sci 1964, 42:465-482.
117. Cecilia D, Gadkari DA, Kedarnath N, and Ghosh SN. Epitope mapping of Japanese encephalitis virus envelope protein using monoclonal antibodies against an Indian strain. J Gen Virol 1988, 69:2741-2747.
118. Peiris JSM, and Porterfield JS. Antibody-mediated enhancement of Flavirirus replication in macrophage-like cell lines. Nature 1979, 282:509-511.
119. Cardosa MJ, Porterfield JS, and Goron S. Complement receptor mediates enhanced flavivirus replication in macrophages. J Exp Med 1983, 158:258-263.
120. Schlesinger JJ, and Brandriss MW. Growth of 17D yellow fever rivus in a macrophage-like cell line, U937: role of Fc and viral receptors in antibody-mediated infection. J Immunol 1981, 127:659-665.
121. Barrett ADT, and Gould, EA. Antibody-mediated early death *in vivo* after infection with yellow fever virus. J Gen Virol 1986, 67:2539-2542.
122. Phillpotts RJ, Stephenson JR, and Porterfield JS. Antibody-mediated enhancement of tick-borne encephalitis virus infectivity. J Gen Virol 1985, 66:1831-1837.
123. Peiris JSM, and Porterfield JS. Antibody-dependent plaque enhancement: its antigenic specificity in relation to Togaviridae. J Gen Virol 1982, 58:291-296.
124. King AA, Sands JJ, and Porterfield JS. Antibody-mediated enhancement of rabies virus infection in a mouse macrophage cell line (P388D1). J Gen Virol 1984, 65:1092-1093.
125. Chanas AC, Gould EA, Clegg JCS, and Varma MGR. Monoclonal antibodies to Sindbis virus glycoprotein E1 can neutralize, enhance infectivity, and independtly inhibit haemagglutination or haemolysis. J Gen Virol 1982, 58:37-46.
126. Vennema H, deGroot RJ, Harbour DA, Dalderup M, Gruffydd-Jones T, Horzinek MC, and Spann WJM. Early death after feline infectious peritonitis virus challenge due to recomginant vaccinia virus immunization. J Virol 1990, 64:1407-1409.
127. Chin J, Magoffin RL, Shearer LA, Schieble J H, and Lennette Eh. Field evaluation of a respiratory syncytial virus vaccine and a trivalent parainfluenza virus vaccine in a pediatric population. Am J Epidemiol 1969, 89:449-463.
128. Fulginiti VA, Eller JJ, Sieber OF, Joyner JW, Minamitani M, and Meiklejohn G. Respiratory virus immunization I. A field trial of two inactivated respiratory virus vaccines; and aqueous trivalent parainfluenza virus vaccine and an alum-precipated respiratory syncytial virus vaccine. Am J Epidemiol 1969, 89:435-448.
129. Kim HW, Canchola JG. Brandt Cd, Pyles G, Chanock RM, Jensen K, and Parrott RH. Respiratory syncytial virus disease in infants despite prior administration of antigenic inactivated vaccine. Am J Epidemiod 1969, 89:422-434.
130. Kapikian AZ, Mitchell RH, Chanock RM, Shvedoff RA, and Stewart, CE. An epidemiologic study of altered clinical reactivity to respiratory syncytial (RS) virus infection in children previously vaccinated with an inactivated RS virus vaccine. Am J Epidemiol 1969, 89:405-421.
131. Prince GA, Jenson AB, Hemming VG, Murphy BR, Walsh EE, Horswood RL, and Chanock RM. Enhancement of respiratory syncytial virus pulmonary pathology in cotton rats by prior intramuscular inoculation of formalin-inactivated virus. J Virol 1986, 57:721-728.
132. Fulginiti VA, Eller JJ, Downie AW, and Kempe CH. Altered reactrivity to measles virus: atypical measles in children previously immunized with inactivated measles virus vaccines. J Am Med Assoc 1967, 202:1075-1080.
133. Rauh LW, and Schmidt R. Measles immunization with killed virus vaccine: serum antibody titers and experience with exposure to measles epidemic. Amer J Dis Child 1965, 109:232-237.
134. McGuire TC, Adams DS, Johnson GC, Klevjer-Anderson P, Barbee DD, and Gorham JR. Acute arthritis in caprine arthritis-encephalitis virus challenge exposure of vaccinated or persistently infected goats. Am J Vet Res 1986, 47:537-540.
135. Knowles D Jr, Cheevers W, McGuire T, Stem T, and Gorham J. Severity of arthritis is predicted by antibody response to gp135 in chronic infection iwth caprine arthritis-encephalitis virus. J Virol 1990, 64:2396-2398.
136. Robinson WE Jr, Montefiori DC, and Mitchell WM. A human immunodeficiency virus type 1 (HIV-1) infection-enhancing factor in seropositive sera. Biochea Biophys Res Commun 1987, 149:693-699.
137. Robinson WE Jr, Montefiori DC, and Mitchell WM. Antibody-dependent enhancement of human immunodeficienty virus type 1 infection. Lancet 1988, i:790-794.
138. Robinson WE Jr, Montefiori DC, Gilespie DH, and Mitchell WM. Complement-mediated, antibody-dependent enhancement of HIV-1 infection *in vitro* is characterized by increased protein and RNA synthesis and infectious virus release. J Acq Imm Def Syn 1989, 2:33-42.
139. Robinson WE Jr, Montefiori CD, and Mitchell WM. Complement-mediated antibody-dependent enhancement of HIV-1 infection requires CD4 and complement receptors. Virology 1990, 175:600-604.
140. Homsy J, Tateno M, and Levy JA. Antibody-dependent enhancement of HIV infection. Lancet 1988, i:1285-1286.
141. June RA, Schade SZ, Bankowski MJ, Kuhns M, McNamara A, Lint RF, Landay AL, and Spear GT. Complement and antibody mediate enhancement of HIV infaction by increasing virus binding and provirus formation. AIDS 1991, 5:269-274.
142. June RA, Landay AL, Stefanik K, Lint TF, and Spear GT. Phenotypic analysis of complement receptor 2+ T lymphocytes: reduced expression on CD4+ cells in HIV-infected persons. Immunology 1992, 75:59-65.
143. Robinson WE Jr, Kawamura T, Gorny MK, Montefiori DC, Mitchell Wm, Luke D, Matsumoto Y, Sugano T, Masuho Y, Hersh E, and Zolla-Pazner S. Human monoclonal antibodies to the human immunodeficiency virus type 1 (HIV-1) transmembrane glycoprotein gp41 enhance HIV-1 infection *in vitro.* Proc Natl Acad Sci USA 1990, 87:3185-3189.
144. Robinson WE Jr, Kawamura T, Lake D, Masuho Y, Mitchell Wm, and Hersh EM. Entibodies to the primary immunodominant domain of human immunodeficiency virus type 1 (HIV-1) glycoprotein 41 enhance HIV-1 infection Iin vitroI. J Virol 1990, 64:5301-5305.
145. Robinson WE Jr, Gorny MK, Xu, J-Y, Mitchell WM, and Zolla Pazner SA. Two immunodominant domains of gp41 bind antibodies which enhance HIV-1 infection in vitro. J Virol 1991, 65:4169-4176.
146. Klasse PJ, Pipkorn R, and Blomberg J. Presence of antibodies to a putatively immunosuppressive part of human immunodeficiency virus (HIV) envelope glycoprotein gp41 is strongly associated with health among HIV-positive subjects. Proc Natl Acad Sci USA 1988, 85:5225-5229.
147. Gnann JW Jr, Nelson JA, and Oldstone MBA> Fine mapping of an immunodominant domain in the transmembrane glycoprotein of human immunodeficiency virus. J Virol 1987, 61:2639-2641.
148. Chiodi FA, Gegerfeldt J, Albert EM, Fenyö EM, Gaines H, von Sydow M, Biberfeld G, Parks E, and Norrby E. Site directed ELISA with synthetic peptides representing the HIV transmembrane glycoprotein. J Med Virol 1987, 23:1-9.
149. Moore JP, Jameson BA, Weiss RA, and Sattentau QJ. The HIV-cell fusion reaction. In: *Viral Fusion Mechanisms,,* Bentz, J., ed., CRC Press, Boca Raton, FL, 1993, 233-289.
150. Mitchell WM, Torres J, Johnson PR, Hirsch V, Yilma T, Garnder MB, and Robinson WE Jr. Antibodies ot the putative SIV infection-enhancing domain diminish beneficial effects of an SIV gp160 vaccine in rhesus macaques. AIDS 1995, IN PRESS.
151. Mills KH, Page M, Chan WL, Kitchin P, Stott EJ, Taffs F, Jones W, Rose J, Ling C, and Silvera P. Protection against SIV infection in macaques by immunization with inactivated virus from the BK28 molecular clone but not with BK28-derived recombinant env and gag proteins. J Med Primatol 1992, 21:50-58.
152. Giavedoni LD, Planelles V, Haigwood NL, Ahmad S, Kluge JD, Marthas ML, Gardner MB, Luciw PA, and Yilma TD. Immune response of rhesus macaques to recombinant simian immunodeficiency virus gp130 does not protect from challenge infection. J Virol 1993, 67:577-583.
153. Berman PW, Gregory TJ, Riddle L, Nakamura GR, Champe MA, Porter JP, Wurm FM, Hershberg RD, Cobb EK, and Eichberg JW. Protection of chimpanzees from infection after vaccination with recombinant glycoprotein gp120 but not gp 160. Nature 1990, 345:622-625.
154. Hu SL, Fultz PN, McClure HM, Eichberg JW, Thomas EK, Zarling J, Singhal MC, Kosowski SG, Swenson RB, Anderson DC, and Todaro G. Effect of immunization iwth a Vaccinia-HIV env recombinant on HIV infection of chimpanzees. Nature 1987, 328:721-721.
155. Berman PW, Groopman JR< Gregory T, Clapham PR, Weiss RA, Ferriani R, Riddle L, Shimasaki C, Lucas C, Lasky LA, and Eichberg JW. Human immunodeficiency virus tyhpe 1 challenge of chimpanzees immunized with recombinant envelope glycoprotein gp120. Proc Natl Acad Sci USA 1988, 85:5200-5204.
156. Wang SZ, Rushlow KE, Issel CJ, Cook RF, Cook SJ, Raabe ML, Chong Y-H, Costa L, and Montelaro RC. Enhancement of EIAV replication and disease by immunization with a baculovirus-expressed recombinant envelope surface glycoprotein. Virology 1994, 199:2347-251.
157. Gardner MB, Rosenthal A, Jennings M, Yee J, Antipa L, and MacKenzi M. Passiv eimmunization of macaques against SIV infection. J Med Primatol 1994, in press.
158. Robinson J We, Torres JV, and Gardner M. Antibodies to amino acid 603-622 of simian immunodeficiency virus (SIV) transmembrane glycoprotein apparently enhance SIV infection in rhesus macaques. *First National Conference on Human Retroviruses and Related Infections.* Washington, DC, December 1993, abstract 79.
159. Putkonen P, Thortensson R, Ghavamzadeh L, Albert J, Hild K, Biberfeld G, and Norrby E. Prevention of HIV-2 and SIVsm infection by passive immunization in cynomolgus monkeys. Nature 1991, 352:436-548.
160. Hohdatsu, T., Pu R, Torres BA, Trunjillo S, Gardne5r MB, and Yamamoto JC. Passive antibody protection of cats against feline immunodeficiency virus infection. J Virol 1993, 67:2344-2348.
161. Hosie MJ, Osborne R, Reid G, Neil JC, and Jarrett 0. Enhancement after feline immunodeficiency virus vaccination. Vet Immunol Immunopathol 1992, 35:191-197.
162. Kent KA, Kitchen P, Mills KHG, Page M, Taffs F, Corcoran T, Silvera P, Flanagan B, Powell C, Rose J, Ling C, Aubertin AM, and Stott EJ. Passive immunization of cynomolgus macaques with immune sera or a pool of neutralizing monoclonal antibodies failed to protect aginst challenge with SIV_{mac251}. AIDS Res Hum Retroviruses 1994, 10:189-194.
163. Daar ES, Moudgil T, Meyer Rd, and Ho DD. Transient high levels of virema in patients with primary human immunodeficienty virus type 1 infection. N Engl J Med 1991, 324:961-964.
164. Clark SJ, Saag MS, Decker WD, Campbell-Hill S, Roberson JL, Veldkamp PJ, Kappes JC, Hahn B, and Shaw GM. High titers of cytopathic virus in plasma of patients with symptomatic primary HIV-1 infection. N Engl J Med 1991, 324:954-960.
165. Boucher CAB, Lange JMA, Miedema FF, Weverling GJ, Koot M, Mulder JW, Goudsmit J, Kellam P, Larder BA, and Tersmette M. HIV-1 biological phenotype and the devleopment of zidovudine resistance in relation to disease progression in asymptomatic individuals during treatment. AIDS 1992, 6:1259-1264.
166. Taylor JGM, Schwartz K, and Detels R. The time from infection with human immunodeficiency virus (HIV) to the onset of AIDS. J Infect Dis 1986, 154:694:697.
167. Roos MTL, Lange JMA, de Goede REY, Coutinho RA, Schellekens PTA, Miedema F, and Tersmette M. Viral phenotype and immune response in preimary human immunodeficiency virus type I infection. J Infect Dis 1992, 165:427-432.
168. Bozzette SA, McCutchan JA, Spector SA, Wright B, and Richman DD. A cross-sectional comparison of persons with syncytium- and non-syncytium-inducing human immunodeficiency virus. J Infect Dis 1993, 168:1374-1379.
169. Koot M, Keet IPM, Vos AHV, Doede REY, Roos MthL, Coutinho RA, Miedema F, Schellekens PthA, and Tersmette M. Prognostic value of HIV-1 syncytium-inducing phenotype for rate of CD4+ cell depletion and progression to AIDS. Ann Int Med 1993, 118:681-688.
170. Nielsen C, Pedersen C, Lundgren JD, and Gerstoft J. Biological properties of HIV isolates in primary HIV infection: consequences for the subsequent course of infection: consequences for the subsequent course of infection. AIDS 1993, 7:1035-1040.
171. Zhu T. Mo H, Wang N, Nam DS, Cao Y, Loup RA, and Ho DD. Genotypic and phenotypic characterization of HIV-1 in patients with primary infection. Science 1993, 261:1179-1181.
172. Gabriel JL, and Mitchell WM. Proposed atomic structure of atruncated human immunodeficiency virus glycoprotein gp120 derived by molecular modeling: targer CD4 recognization and docking mechanism. Proc Natl Sci USA 1993, 90:4186-4190.
173. LaRosa GJ, Davide JP, Weinhold K, Waterbury JA, Profy AT, Lewis JA< Langlois AJ, Dreesman GR, Boswell RN, Shadduck P, Holley LH, Karplus M, Bolognesi DP, Matthews TJ, Emini EA, and Putney SD. Conserved sequence and structural elements in the HIV-1 principal neutralization determinant. Science 1990, 249:932-935.
174. Leonard CK, Spellman MW, Riddle L, Harris RJ, Thomas JN, and Gregor TJ. Assignment of intrachain disulfide bonds and characterization of potential glycosylation sites of the type 1 recombinant human immunodeficienty virus envelope glycoprotein (gp120) expressed in Chinese hamster ovary cells. J Biol Chem 1990, 265:10373-10382.
175. Ebenbichler C, Westervelt P, Carrillo A, Henkel T, Johnson D, and Ratner L. Structure-function relationships of the HI-1 envelope V3 loop tropism determinant: evidence for two distinct conformations. AIDS 1991, 5:1-14.
176. Gu R, Westervelt P, and Ratner L. Role of HIV-1 envelope V3 loop cleavage in cell tropism. AIDS Res Hum Retrovir 1993, 9:1107-1015.
177. Tindall B, and Cooper DA. Primary HIV infection: host responses and intervention strategies. AIDS 1991, 5:1-14.
178. Robinson WE jr, and Mitchell WM. Neutralization and enhancement of *in vitro* and *in vivo* HIV and SIV infection. AIDS 1990, 4:S151-S162.
179. Gabrien JL, and Mitchell Wm. GP120 docking interactions and inhibition design based on an atomic structure derived by molecular modeling using th eDreiding II Force Field. Proceedings of the Distance-based approaches to protein structure determination II Symposium, 1994, in press.
180. Freed E, Myers JD, and Risser R. Mutational analysis of the cleavage sequence of the human immunodeficiency virus type 1 envelope glycoprotein precursor gp160. J Virol 1989, 63:4670-4675.
181. Pinter A, Honen WJ, Tilley SA, Bona C, Zaghouani H, Gorney MK, and Zolla-Pazner S. Oligomeric structure of gp41, the transmembrane protein of human immunodeficiency virus type 1. J Virol 1989, 63:2674-2679.
182. Chada S, De JeSus CE, Townsend K, Lee WTL, Laube L, Jolly DJ, Change SMW, and Warner JF. Cross-reactive lysis of human targets infected with prototypic and clinical Huna Immunodeficiency Virus Type (HIV-1) strains by murine auto-HIV-1 IIIB env-specific cytotixic T lymphocytes. J Virol 1993, 67:3409-3417.
183. Aslandis C, and DeJong PU. Ligation-independent cloning of PCR products (LIC-PCR). Nuc Acid Res 1990, 18:6069-6074.
184. Haun RA, Servanti IM, and Moss J. Rapid, reliable Ligation-independent cloning of PCR products using modified plasma vectors. BioTechniques 1992, 13:515-518.
185. Montefiori DC, Robinson WE, Schuffman SS, and Mitchell WM. Evaluation of antiviral drugs and neutralizing antibodies against human immunodeficiency virus by a rapid and sensitive microtiter infectin assay. J Clin Microbiol 1988, 26:231-235.
186. Dalgleish AG, Chanh TC, Kennedy RC, Kanda P, Clapham PR and Weiss RA. Neutralization of diverse HIV-1 strains by nomoclonal antibodies raised against a gp41 synthetic peptide. Virology 1988, 165:209-215.
187. Mitchell WM, and Rosenbloom T. Induction of mucosal and humoral anti-HIV responses iwth HIV env-DNA. Keystone Symposium on Mucosal Immunity: New Strategies for Protection Against Viral and Bacterial Pathogens. Jan 1995, Keystone, CO.
188. Sambrook *et al. Molecular Cloning: A Laboratory Manual,* 2nd ED., Cold Spring Harbor Laboratory, Cold Spring Harbor, New York, 1989
189. *Fundemental Virology, 2nd Ed*. Bernard N. Fields and David M. Knipe, Chief Eds., Raven Press:New York, 1990
190. Martin, E.W. (ed.) *Remington's Pharmaceutical Sciences,* latest edition Mack Publishing Co., Easton, PA
191. *Mechanisms* of *Microbial Disease, 2nd E.*. Moselio Schaechter, Gerald Medoff and Barry I, Eisenstein, Eds., Williams & Wilkins, Baltimore, 1993.
192. Ni J., Porter, G.A. and Hollander, D. "β7 Integrins and Other Cell Adhesion Molecules Are Differentially Expressed and Modulated by TNFβ in Different Lymphocyte Populations" *Cell Immunol*. 1995, 161:166-172.

## Claims

1. Use of vitamin D3 and an antigen-encoding DNA, wherein the DNA is complexed to a transfection-facilitating cationic lipid in the manufacture of a medicament for inducing a mucosal immune response.

2. The use of claim 1, wherein the cationic lipid is lipospermine, lipospermidine or dioctadecylamidoglycylspermine.

3. The use of claim 1 or 2, wherein the DNA encodes an envelope antigen or envelope-associated antigen.

4. The use of claim 3, wherein the DNA encodes an envelope antigen.

5. The use of any of claims 1-4, wherein the vitamin D3 is 1,25(OH)₂D3.

6. A composition comprising vitamin D3 and an antigen-encoding DNA, wherein the DNA is complexed to a transfection-facilitating cationic lipid.

7. The composition of claim 6, wherein the cationic lipid is lipospermine, lipospermidine or dioctadecylamidoglycylspermine.

8. The composition of claim 6 or 7, wherein the DNA encodes an envelope antigen or envelope-associated antigen.

9. The composition of claim 8, wherein the DNA encodes an envelope antigen.

10. The composition of any of claims 7-10, wherein the vitamin D3 is 1,25(OH)₂D3.

## Patentansprüche

1. Verwendung von Vitamin B3 und einer Antigen kodierenden DNS, wobei die DNS mit einem die Transfektion fördernden kationischen Lipid komplexgebunden ist, zur Herstellung eines Medikamentes zum Induzieren einer mukosalen Immnantwort.

2. Verwendung nach Anspruch 1, wobei das kationische Lipid aus der Gruppe Lipospermin, Lipospermidin oder Dioctadecylamidoglycylspermin gewählt ist.

3. Verwendung nach Anspruch 1 oder 2, wobei die DNS ein Envelope Antigen oder ein Envelop-assoziiertes Antigen kodiert.

4. Verwendung nach Anspruch 3, wobei die DNS ein Envelope Antigen kodiert.

5. Verwendung nach einem der Ansprüche 1 bis 4, wobei das Vitamin B3 1,25(OH)₂D3 ist.

6. Zusammensetzung, welche Vitamin B3 und eine Antigen kodierende DNS enthält, wobei die DNS mit einem die Transfektion fördernden kationischen Lipid komplexgebunden ist.

7. Zusammensetzung nach Anspruch 6, wobei das kationische Lipid aus der Gruppe Lipospermin, Lipospermidin oder Dioctadecylamidoglycylspermin gewählt ist.

8. Zusammensetzung nach Anspruch 6 oder 7, wobei die DNS ein Envelope Antigen oder ein Envelop-assoziiertes Antigen kodiert.

9. Zusammensetzung nach Anspruch 8, Verwendung nach Anspruch 3, wobei die DNS ein Envelope Antigen kodiert.

10. Zusammensetzung nach einem der Ansprüche 7 bis 10, wobei das Vitamin B3 1,25(OH)₂D3 ist.

## Revendications

1. Utilisation de la vitamine D3 et d'un ADN codant pour un antigène, dans laquelle l'ADN est combiné à un lipide cationique facilitant la trarisfection, pour la fabrication d'un médicament induisant une réponse mucosal-immune

2. Utilisation selon la revendication 1 dans laquelle le lipide cationique est la lipospermine, la lipospermidine ou la dioctadecylamidoglycylspermine

3. Utilisation selon la revendication 1 ou 2 dans laquelle l'ADN code pour un antigène enveloppe ou un antigène enveloppe-associé

4. Utilisation selon la revendication 3 dans laquelle l'ADN code pour un antigène enveloppe

5. Utilisation selon l'une quelconque des revendications 1 à 4 dans laquelle la vitamine D3 est 1,25(OH)₂D3

6. Composition comprenant la vitamine D3 et un ADN codant pour un antigène, dans laquelle l'ADN est combiné à un lipide cationique facilitant la trarisfection

7. Composition selon la revendication 6 dans laquelle le lipide cationique est la lipospermine, lipospermidine ou la dioctadecylamidoglycylspermine

8. Composition selon la revendication 6 ou 7 dans laquelle l'ADN code pour un antigène enveloppe

9. Composition selon la revendication 8 dans laquelle l'ADN code pour un antigène enveloppe

10. Composition selon l'une quelconque des revendications 7 à 10 dans laquelle la vitamine D3 est 1,25(OH)₂D3
